# EUROPEAN PATENT SPECIFICATION

(11) **EP 4 460 232 B1**
(45) Date of publication and mention of the grant of the patent: **04.11.2026**
(21) Application number: 23704016.7
(22) Date of filing: 05.01.2023
(51) Int. Cl.: A61B 5/145, A61B 5/00

(54) **WEARABLE ANALYTE MONITORING DEVICE**
AM KÖRPER TRAGBARE ANALYTÜBERWACHUNGSVORRICHTUNG
DISPOSITIF PORTABLE DE SURVEILLANCE D'ANALYTE

(30) Priority: 05.01.2022 US 202263296830 P
(43) Date of publication of application: 13.11.2024
(73) Proprietor: Biolinq Incorporated, San Diego, CA 92121 (US)
(72) Inventor: BRISTER, Mark Christopher, San Diego, California 92130 (US); MANSFIELD III, George Albert, San Diego, California 92122 (US); MORELOCK, David Michael, Escondido, California 92029 (US); GOTTLIEB, Emily, San Diego, California 92121 (US); HAROUSH, Tsuk, San Diego, California 92121 (US); PATEL, Sonny, San Diego, California 92121 (US)
(74) Representative: Cooley (UK) LLP
(86) International application number: PCT/US2023/060177
(87) International publication number: WO 2023/133468

(56) References cited:
- AU-B2- 2015 227 515
- US-A1- 2021 000 399
- US-A1- 2021 379 370

## Description

### CROSS-REFERENCE TO RELATED APPLICATIONS

This application claims priority to U.S. Provisional Patent Application No. 63/296,830, filed January 5, 2022.

### TECHNICAL FIELD

This invention relates generally to the field of analyte monitoring, such as continuous glucose monitoring.

### BACKGROUND

Diabetes is a chronic disease in which the body does not produce or properly utilize insulin, a hormone that regulates blood glucose. Insulin may be administered to a diabetic patient to help regulate blood glucose levels, though blood glucose levels must nevertheless be carefully monitored to help ensure that timing and dosage are appropriate. Without proper management of their condition, diabetic patients may suffer from a variety of complications resulting from hyperglycemia (high blood sugar levels) or hypoglycemia (low blood sugar levels).

Blood glucose monitors help diabetic patients manage their condition by measuring blood glucose levels from a sample of blood. For example, a diabetic patient may obtain a blood sample through a fingerstick sampling mechanism, transfer the blood sample to a test strip with suitable reagent(s) that react with the blood sample, and use a blood glucose monitor to analyze the test strip to measure glucose level in that blood sample. However, a patient using this process can typically only measure his or her glucose levels at discrete instances in time, which may fail to capture a hyperglycemia or hypoglycemia condition in a timely manner. Yet a more recent variety of glucose monitor is a continuous glucose monitor (CGM) device, which includes implantable transdermal electrochemical sensors that are used to continuously detect and quantify blood glucose levels by proxy measurement of glucose levels in the subcutaneous interstitial fluid. However, conventional CGM devices also have weaknesses including tissue trauma from insertion and signal latency (e.g., due to the time required for the glucose analyte to diffuse from capillary sources to the sensor). These weaknesses also lead to a number of drawbacks, such as pain experienced by the patient when electrochemical sensors are inserted, and limited accuracy in glucose measurements, particularly when blood glucose levels are changing rapidly. Accordingly, there is a need for a new and improved analyte monitoring system. AU2015227515B2 discloses a microneedle-based delivery system comprising a housing, a biasing element and an actuator for deploying a microneedle array into the skin, thus representing similar background technology for wearable analyte monitoring devices.

### SUMMARY

The invention is as defined in the appended claims. According to an embodiment, the present disclosure relates to analyte monitoring.

In embodiments, the present disclosure further relates to a wearable analyte monitoring device, comprising a housing comprising a body defining a cavity therein, wherein the housing body comprises a distal opening; an adhesive layer coupled to a distal end of the housing and surrounding the distal opening, the adhesive layer configured to secure the device to a skin surface of the user, a biasing element contained within the cavity, a microneedle array coupled to the biasing element and comprising a plurality of microneedles, a retention element contained within the cavity and configured to releasably retain the biasing element, and an actuation member coupled to the retention element, wherein engagement of the actuation member moves the microneedle array between a first configuration and a second configuration, and wherein in the first configuration, the microneedle array is held within the cavity of the housing body, and in the second configuration, the microneedle array protrudes through the distal opening of the housing body.

In embodiments, the present disclosure further relates to a method of inserting a microneedle array into a skin surface of a user, the method comprising providing a wearable analyte monitoring device comprising the microneedle array in a first configuration, the microneedle array comprising a plurality of microneedles, the microneedle array coupled to a biasing element contained within a cavity of a housing, the housing comprising a body defining the cavity therein, the biasing element releasably retained by a retention element contained within the cavity, and the retention element coupled to an actuation member, and transitioning the microneedle array from the first configuration to a second configuration, and wherein in the first configuration, the microneedle array is held within the cavity of the housing body, and in the second configuration, the microneedle array protrudes through a distal opening of the housing body.

In embodiments, the present disclosure further relates to an analyte monitoring device, comprising a housing comprising a body defining a cavity therein, wherein the housing body comprises a distal opening, a biasing element contained within the cavity, a microneedle array coupled to the biasing element, and an actuation member, wherein engagement of the actuation member moves the microneedle array from a first configuration to a second configuration under influence of the biasing element, and wherein in the first configuration, the microneedle array is held within the cavity of the housing body, and in the second configuration, at least a portion of the microneedle array protrudes through the distal opening of the housing body.

In embodiments, the present disclosure further relates to a method of monitoring a user using a wearable analyte monitoring device, the method comprising providing the wearable analyte monitoring device comprising the microneedle array in a first configuration, the microneedle array comprising a plurality of microneedles, the microneedle array coupled to a biasing element contained within a cavity of a housing, the housing comprising a body defining the cavity therein, the biasing element releasably retained by a retention element contained within the cavity, and the retention element coupled to an actuation member, adhering the wearable analyte monitoring device to a skin surface of the user, transitioning the microneedle array from the first configuration to a second configuration, and wherein in the first configuration, the microneedle array is held within the cavity of the housing body, and in the second configuration, the microneedle array protrudes through a distal opening of the housing body, and measuring a target analyte level in dermal interstitial fluid of the subject with the microneedle array.

In embodiments, the present disclosure further relates to a method of inserting a microneedle array into a skin surface, the method comprising providing the microneedle array within a cavity of a housing, the housing comprising a body defining the cavity therein, wherein the microneedle array is coupled to a biasing element within the cavity, loading the microneedle array in first configuration in which the microneedle array is biased by the biasing element toward a distal end of the housing body, and providing an actuation member, wherein the actuation member is engaged to release the microneedle array from the first configuration and transition the microneedle array to a second configuration in which a plurality of microneedles of the microneedle array protrude from a distal opening of the housing body, wherein in the transition from the first configuration to the second configuration, the microneedle array travels within the cavity toward the distal end of the housing body under influence of the biasing element.

### BRIEF DESCRIPTION OF THE DRAWINGS

FIG. 1 depicts an illustrative schematic of an analyte monitoring system with a microneedle array.
FIG. 2A depicts an illustrative schematic of an analyte monitoring device.
FIG. 2B depicts an illustrative schematic of microneedle insertion depth in an analyte monitoring device.
FIGS. 3A-3D depict an upper perspective view, a side view, a bottom view, and an exploded view, respectively, of an analyte monitoring device.
FIGS. 4A-4E depict a perspective exploded view, a side exploded view, a lower perspective view, a side view, and an upper perspective view, respectively, of a sensor assembly in an analyte monitoring device.
FIGS. 4F-4H depict a perspective exploded view, a side exploded view, and a side view, respectively, of a sensor assembly in an analyte monitoring device.
FIG. 5A depicts an illustrative schematic of a microneedle array. FIG. 5B depicts an illustrative schematic of a microneedle in the microneedle array depicted in FIG. 5A.
FIG. 6 depicts an illustrative schematic of a microneedle array used for sensing multiple analytes.
FIG. 7A depicts a cross-sectional side view of a columnar microneedle having a tapered distal end. FIGS. 7B and 7C are images depicting perspective and detailed views, respectively, of an embodiment of the microneedle shown in FIG. 7A.
FIG. 8 depicts an illustrative schematic of a columnar microneedle having a tapered distal end.
FIGS. 9A and 9B depict illustrative schematics of a microneedle array and a microneedle, respectively. FIGS. 9C-9F depict detailed partial views of an illustrative variation of a microneedle.
FIGS. 10A and 10B depict an illustrative variation of a microneedle.
FIGS. 11A and 11B depict illustrative schematics of a microneedle array configuration. FIGS. 11C and 11D depict illustrative schematics of a microneedle array configuration.
FIGS. 12A and 12B depict perspective and orthogonal views, respectively, of an illustrative variation of a die including a microneedle array.
FIGS. 13A-13E depict illustrative schematics of different variations of microneedle array configurations.
FIGS. 14A-14G depict illustrative schematics of aspects of a wearable analyte monitoring device.
FIGS. 15A-15E depict illustrative schematics of aspects of a wearable analyte monitoring device.
FIGS. 16A-16C depict illustrative schematics of aspects of a wearable analyte monitoring device.
FIGS. 17A-17E depict illustrative schematics of aspects of a wearable analyte monitoring device.
FIGS. 18A-18C depict illustrative schematics of aspects of a wearable analyte monitoring device.
FIGS. 19A-19B depict illustrative schematics of aspects of a wearable analyte monitoring device.

### DETAILED DESCRIPTION

Non-limiting examples of various aspects and variations of the invention are described herein and illustrated in the accompanying drawings.

Aspects of the current subject matter are directed to a microneedle array-based analyte monitoring device with integrated applicator. In some variations, an integrated applicator mechanism allows for a user to place the analyte monitoring device on the desired area and deploy the microneedle array to puncture the skin of the user for insertion into the skin. In some variations, the analyte monitoring device is secured to the skin at the desired area via an adhesive prior to the microneedle array being deployed.

In some variations, the microneedle array-based analyte monitoring device (also referred to herein as an analyte monitoring device, a wearable analyte monitoring device, and/or a wearable analyte monitoring device with integrated applicator) transitions a microneedle array from a first configuration to a second configuration. In some variations, the first configuration is a loaded configuration (e.g., when the microneedle array is in the first configuration, the analyte monitoring device and/or a biasing element are loaded such that the microneedle array is ready to be deployed), and the second configuration is a deployed configuration (e.g., when the microneedle array is in the second configuration, the analyte monitoring device and/or a biasing element are deployed such that the microneedle array is inserted into the skin of the user). In the first configuration, the microneedle array is retained inside the housing of the wearable analyte monitoring device away from the surrounding electronics and housing components. By retaining the microneedle array within the housing in the first configuration, the plurality of microneedles of the microneedle array may be protected from damage prior to deployment. This arrangement allows for the microneedle array to travel (e.g., transition to a deployed or second configuration) in a generally vertical direction independent of the supporting electronics and housing. By isolating or separating the microneedle array from other components, the mass of a support structure holding the microneedle array is low, enabling rapid acceleration of the microneedle array over a relatively small displacement with relatively small forces when compared to moving an entire device body (e.g., as would be required with a separate applicator device). This arrangement minimizes impact momentum, which reduces discomfort to the user on impact. The reduced moving mass also enables reduction in spring size and required spring force to the degree that components for effective insertion are small enough to fit inside of a wearable sensor body housing.

In variations, upon assembly of the analyte monitoring device, the microneedle array is positioned in a first, loaded configuration, in which it is retracted inside a housing body and held in the first configuration by a retention element (such as a movable clip) that can be dislocated by an actuation member from, for example, an exterior of the housing body. In the first configuration, a biasing element is compressed into a stressed state and presses on the microneedle array with a force (e.g., between about 15 to about 35 Newtons). Once the biasing element is released from the retention element via actuation by the user, the biasing element applies an accelerating force on the microneedle array in the direction of application. Due to the small mass, the force accelerates the microneedle array to relatively high speeds (e.g., between about 7 to about 14 m/s) in a very short displacement distance (e.g., between about 1.5 to about 3 mm) to impact the skin. The speeds overcome the viscoelastic mechanical properties of the skin surface, thus effectively and reliably inserting the microneedle array.

In some variations, the microneedle array maintains electrical connectivity with the electronics of the analyte monitoring device via a mechanically flexible connection. In some variations, an electrical connection is established with the electronics of the analyte monitoring device when the microneedle array reaches the deployed or second configuration in which the microneedle array protrudes from a distal opening for insertion into the skin of the user. In some variations, a seal is maintained between the microneedle array and the housing while transitioning from the first configuration to the second configuration. In some variations, a seal is established when the microneedle array reaches the deployed or second configuration.

Before providing additional details regarding aspects of the wearable analyte monitoring device with integrated applicator, the following provides a description of some examples of an analyte monitoring device that may be used with the wearable analyte monitoring device described herein. The following descriptions are meant to be exemplary, and aspects related to the wearable analyte monitoring device with integrated applicator consistent with the current subject matter are not limited to the example analyte monitoring device described herein.

As generally described herein, an analyte monitoring system may include an analyte monitoring device that is worn by a user and includes one or more sensors for monitoring at least one analyte of a user. The sensors may, for example, include one or more electrodes configured to perform electrochemical detection of at least one analyte. The analyte monitoring device may communicate sensor data to an external computing device for storage, display, and/or analysis of sensor data.

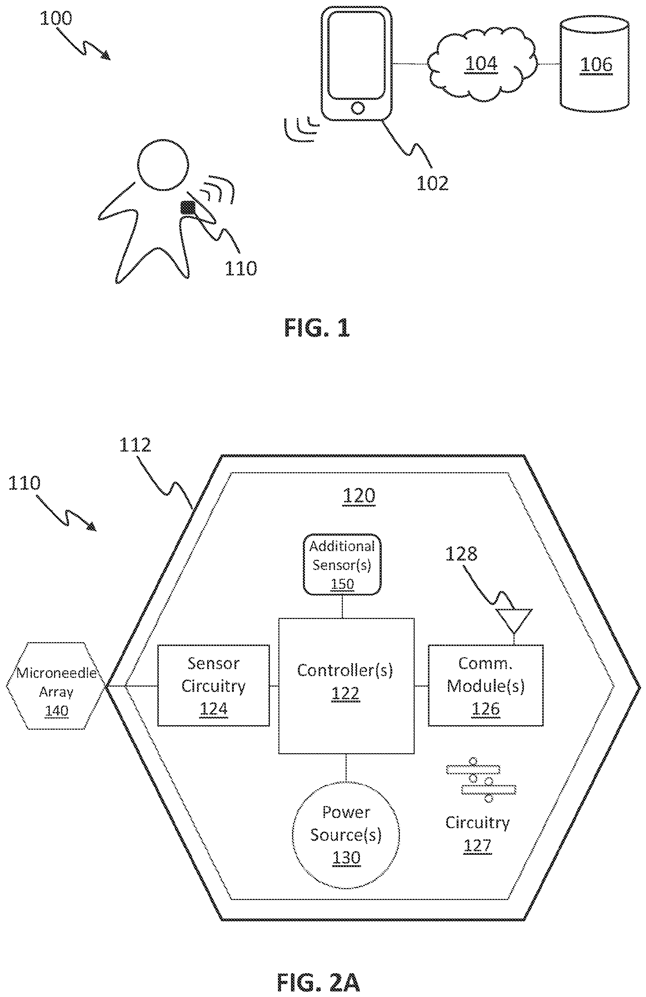

For example, as shown in FIG. 1, an analyte monitoring system 100 may include an analyte monitoring device 110 that is worn by a user, and the analyte monitoring device 110 may be a continuous analyte monitoring device (e.g., continuous glucose monitoring device). The analyte monitoring device 110 may include, for example, a microneedle array comprising at least one electrochemical sensor for detecting and/or measuring one or more analytes in body fluid of a user. The analyte monitoring device 110 may include one or more processors for performing analysis on sensor data, and/or a communication module (e.g., wireless communication module) configured to communicate sensor data to a mobile computing device 102 (e.g., smartphone) or other suitable computing device. In some variations, the mobile computing device 102 may include one or more processors executing a mobile application to handle sensor data (e.g., displaying data, analyzing data for trends, etc.) and/or provide suitable alerts or other notifications related to the sensor data and/or analysis thereof. It should be understood that while in some variations the mobile computing device 102 may perform sensor data analysis locally, other computing device(s) may alternatively or additionally remotely analyze sensor data and/or communicate information related to such analysis with the mobile computing device 102 (or other suitable user interface) for display to the user. Furthermore, in some variations the mobile computing device 102 may be configured to communicate sensor data and/or analysis of the sensor data over a network 104 to one or more storage devices 106 (e.g., server) for archiving data and/or other suitable information related to the user of the analyte monitoring device.

The analyte monitoring devices described herein have characteristics that improve a number of properties that are advantageous for a continuous analyte monitoring device such as a continuous glucose monitoring (CGM) device. For example, the analyte monitoring device described herein have improved sensitivity (amount of sensor signal produced per given concentration of target analyte), improved selectivity (rejection of endogenous and exogenous circulating compounds that can interfere with the detection of the target analyte), and improved stability to help minimize change in sensor response over time through storage and operation of the analyte monitoring device. Additionally, compared to conventional continuous analyte monitoring devices, the analyte monitoring devices described herein have a shorter warm-up time that enables the sensor(s) to quickly provide a stable sensor signal following implantation, as well as a short response time that enables the sensors(s) to quickly provide a stable sensor signal following a change in analyte concentration in the user. Furthermore, as described in further detail below, the analyte monitoring devices described herein may be applied to and function in a variety of wear sites, and provide for pain-free sensor insertion for the user. Other properties such as biocompatibility, sterilizability, and mechanical integrity are also optimized in the analyte monitoring devices described herein.

Although the analyte monitoring systems described herein may be described with reference to monitoring of glucose (e.g., in users with Type 2 diabetes, Type 1 diabetes), it should be understood that such systems may additionally or alternatively be configured to sense and monitor other suitable analytes. As described in further detail below, suitable target analytes for detection may, for example, include glucose, ketones, lactate, and cortisol. One target analyte may be monitored, or multiple target analytes may be simultaneously monitored (e.g., in the same analyte monitoring device). For example, monitoring of other target analytes may enable the monitoring of other indications such as stress (e.g., through detection of rising cortisol and glucose) and ketoacidosis (e.g., through detection of rising ketones).

As shown in FIG. 2A, in some variations, an analyte monitoring device 110 may generally include a housing 112 and a microneedle array 140. In some variations, the microneedle array extends outwardly from the housing when in a deployed configuration. The housing 112, may, for example, be a wearable housing configured to be worn on the skin of a user such that the microneedle array 140 extends at least partially into the skin of the user after deployment. For example, the housing 112 may include an adhesive such that the analyte monitoring device 110 is a skin-adhered patch that is simple and straightforward for application to a user. The microneedle array 140 may be configured to puncture the skin of the user and include one or more electrochemical sensors (e.g., electrodes) configured for measuring one or more target analytes that are accessible after the microneedle array 140 punctures the skin of the user. In some variations, the analyte monitoring device 110 may be integrated or self-contained as a single unit, and the unit may be disposable (e.g., used for a period of time and replaced with another instance of the analyte monitoring device 110).

An electronics system 120 may be at least partially arranged in the housing 112 and include various electronic components, such as sensor circuitry 124 configured to perform signal processing (e.g., biasing and readout of electrochemical sensors, converting the analog signals from the electrochemical sensors to digital signals, etc.). The electronics system 120 may also include at least one microcontroller 122 for controlling the analyte monitoring device 110, at least one communication module 126, at least one power source 130, and/or other various suitable passive circuitry 127. The microcontroller 122 may, for example, be configured to interpret digital signals output from the sensor circuitry 124 (e.g., by executing a programmed routine in firmware), perform various suitable algorithms or mathematical transformations (e.g., calibration, etc.), and/or route processed data to and/or from the communication module 124. In some variations, the communication module 126 may include a suitable wireless transceiver (e.g., Bluetooth transceiver or the like) for communicating data with an external computing device 102 via one or more antennas 128. In some variations, one or more antennas 128 of the communication module 126 are configured for near-field communication. For example, the communication module 126 may be configured to provide uni-directional and/or bi-directional communication of data with an external computing device 102 that is paired with the analyte monitoring device 110. The power source 130 may provide power for the analyte monitoring device 110, such as for the electronics system. The power source 130 may include battery or other suitable source, and may, in some variations, be rechargeable and/or replaceable. Passive circuitry 127 may include various non-powered electrical circuitry (e.g., resistors, capacitors, inductors, etc.) providing interconnections between other electronic components, etc. The passive circuitry 127 may be configured to perform noise reduction, biasing and/or other purposes, for example. In some variations, the electronic components in the electronics system 120 may be arranged on one or more printed circuit boards (PCB), which may be rigid, semi-rigid, or flexible, for example. Additional details of the electronics system 120 are described further below.

In some variations, the analyte monitoring device 110 may further include one or more additional sensors 150 to provide additional information that may be relevant for user monitoring. For example, the analyte monitoring device 110 may further include at least one temperature sensor (e.g., thermistor) configured to measure skin temperature, thereby enabling temperature compensation for the sensor measurements obtained by the microneedle array electrochemical sensors.

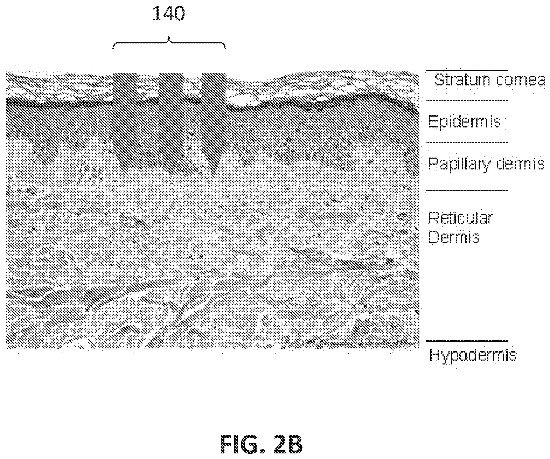

The microneedle array 140 in the analyte monitoring device 110 is configured to puncture skin of a user. As shown in FIG. 2B, when the device 110 is worn by the user, the microneedle array 140 may be deployed to extend into the skin of the user such that electrodes on distal regions of the microneedles rest in the dermis. Specifically, in some variations, the microneedles may be designed to penetrate the skin and access the upper dermal region (e.g., papillary dermis and upper reticular dermis layers) of the skin, in order to enable the electrodes to access interstitial fluid that surrounds the cells in these layers. For example, in some variations, the microneedles may have a height generally ranging between at least 350 µm and about 515 µm. In some variations, in a deployed configuration, one or more microneedles may extend from the housing such that a distal end of the electrode on the microneedle is located less than about 5 mm from a skin-interfacing surface of the housing, less than about 4 mm from the housing, less than about 3 mm from the housing, less than about 2 mm from the housing, or less than about 1 mm from the housing.

In contrast to traditional continuous analyte monitoring devices (e.g., CGM devices), which include sensors typically implanted between about 8 mm and about 10 mm beneath the skin surface in the subcutis or adipose layer of the skin, the analyte monitoring device 110 has a shallower microneedle insertion depth of about 0.25 mm (such that electrodes are implanted in the upper dermal region of the skin) that provides numerous benefits. These benefits include access to dermal interstitial fluid including one or more target analytes for detection, which is advantageous at least because at least some types of analyte measurements of dermal interstitial fluid have been found to closely correlate to those of blood. For example, it has been discovered that glucose measurements performed using electrochemical sensors accessing dermal interstitial fluid are advantageously highly linearly correlated with blood glucose measurements. Accordingly, glucose measurements based on dermal interstitial fluid are highly representative of blood glucose measurements.

Additionally, because of the shallower microneedle insertion depth of the analyte monitoring device 110, a reduced time delay in analyte detection is obtained compared to traditional continuous analyte monitoring devices. Such a shallower insertion depth positions the sensor surfaces in close proximity (e.g., within a few hundred micrometers or less) to the dense and well-perfused capillary bed of the reticular dermis, resulting in a negligible diffusional lag from the capillaries to the sensor surface. Diffusion time is related to diffusion distance according to *t = x*²/(2D) where *t* is the diffusion time, x is the diffusion distance, and D is the mass diffusivity of the analyte of interest. Therefore, positioning an analyte sensing element twice as far away from the source of an analyte in a capillary will result in a quadrupling of the diffusional delay time. Accordingly, conventional analyte sensors, which reside in the very poorly vascularized adipose tissue beneath the dermis, result in a significantly greater diffusion distance from the vasculature in the dermis and thus a substantial diffusional latency (e.g., typically 5 - 20 minutes). In contrast, the shallower microneedle insertion depth of the analyte monitoring device 110 benefits from low diffusional latency from capillaries to the sensor, thereby reducing time delay in analyte detection and providing more accurate results in real-time or near real-time. For example, in some embodiments, diffusional latency may be less than 10 minutes, less than 5 minutes, or less than 3 minutes.

Furthermore, when the microneedle array rests in the upper dermal region, the lower dermis beneath the microneedle array includes very high levels of vascularization and perfusion to support the dermal metabolism, which enables thermoregulation (via vasoconstriction and/or vasodilation) and provides a barrier function to help stabilize the sensing environment around the microneedles. Yet another advantage of the shallower insertion depth is that the upper dermal layers lack pain receptors, thus resulting in a reduced pain sensation when the microneedle array punctures the skin of the user, and providing for a more comfortable, minimally-invasive user experience.

Thus, the analyte monitoring devices and methods described herein enable improved continuous monitoring of one or more target analytes of a user. For example, as described above, the analyte monitoring device may be simple and straightforward to apply, which improves ease-of-use and user compliance. Additionally, analyte measurements of dermal interstitial fluid may provide for highly accurate analyte detection. Furthermore, compared to traditional continuous analyte monitoring devices, insertion of the microneedle array and its sensors may be less invasive and involve less pain for the user. Additional advantages of other aspects of the analyte monitoring devices and methods are further described below.

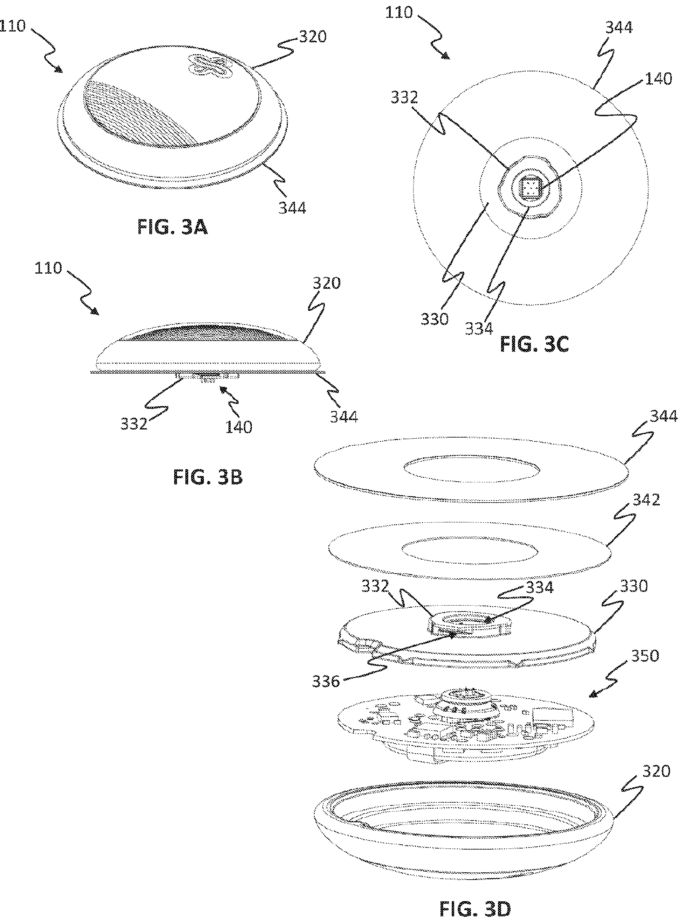

FIG. 3A - FIG. 3D depict aspects of the analyte monitoring device 110. FIGS. 3A-3D depict an upper perspective view, a side view, a bottom view, and an exploded view, respectively, of the analyte monitoring device 110.

The analyte monitoring device 110 may include a housing which defines a cavity that at least partially surrounds or encloses other components (e.g., electronic components) of the analyte monitoring device 110, such as for protection of such components. For example, the housing may be configured to help prevent dust and moisture from entering the analyte monitoring device 110. In some variations, an adhesive layer may be provided at a distal end of the housing to attach the housing to a surface (e.g., skin) of a user. In some variations, after the house is attached to the surface, the microneedle array 140 may be deployed to extend outwardly from the housing and into the skin of the user. Furthermore, in some variations, the housing may generally include rounded edges or corners and/or be low-profile to reduce interference with clothing, etc. worn by the user.

For example, as shown in FIGS. 3A-3D, an example variation of the analyte monitoring device 110 may include a housing cover 320 and a base plate 330, configured to at least partially surround internal components of the analyte monitoring device 110. For example, the housing cover 320 and the base plate 330 may provide an enclosure for a sensor assembly 350 including the microneedle array 140 and electronic components. Once deployed, the microneedle array 140 extends outwardly from a portion of the base plate 330 in a skin-facing direction (e.g., an underside) of the analyte monitoring device 110.

The housing cover 320 and the base plate 330 may, for example, include one or more rigid or semi-rigid protective shell components that may couple together via suitable fasteners (e.g., mechanical fasteners), mechanically interlocking or mating features, and/or an engineering fit. The housing cover 320 and the base plate 330 may include radiused edges and corners and/or other atraumatic features. When coupled together, the housing cover 320 and the base plate 330 may form a cavity comprising an internal volume that houses internal components, such as the sensor assembly 350. For example, the internal components arranged in the internal volume may be arranged in a compact, low-profile stack-up as the sensor assembly 350.

The analyte monitoring device 110 may include one or more adhesive layers provided on a distal end of the housing to attach the analyte monitoring device 110 (e.g., the coupled together housing cover 320 and the base plate 330) to a surface (e.g., the skin) of a user. As shown in FIG. 3D, the one or more adhesive layers may include an inner adhesive layer 342 and an outer adhesive layer 344. The inner adhesive layer 342 may adhere to the base plate 330, and the outer adhesive layer 344 may adhere to the inner adhesive layer 342 and, on its outward facing side, provide an adhesive for adhering (e.g., temporarily) to the skin of the user. The inner adhesive layer 342 and the outer adhesive layer 344 together act as a double-sided adhesive for adhering the analyte monitoring device 110 to the skin of the user. The outer adhesive layer 344 may be protected by a release liner that the user removes to expose the adhesive prior to skin application. In some variations, a single adhesive layer is provided. In some variations, the outer adhesive layer 344, the inner adhesive layer 342, and/or the single adhesive layer may have a perimeter that extends farther than the perimeter or periphery of the housing cover 320 and the base plate 330. This may increase surface area for attachment and increase stability of retention or attachment to the skin of the user. The inner adhesive layer 342, the outer adhesive layer 344, and/or the single adhesive layer may each have an opening that permits passage of the outwardly extending microneedle array 140 when deployed, as further described below. The openings of the inner adhesive layer 342 and the outer adhesive layer 344 may generally align with one another but may, in some variations, differ in size such that one opening is smaller than the other opening. In some variations, the openings are substantially the same size.

The base plate 330 has a first surface (e.g., an outwardly exposed surface) opposite a second surface and serves as a support and/or connection structure and as a protective cover for the sensor assembly 350. The base plate 330 is sized and shaped to attach to the housing cover 320. The base plate 330 may be shaped to securely fit within the housing cover 320 such that outer edges of the base plate 330 align with corresponding edges of an opening of the housing cover 320. The alignment may be such that there is no gap between the outer edges of the base plate 330 and the corresponding edges of the opening of the housing cover 320.

A connection member 332 may be formed in a central or near central region of the first surface of the base plate 330. The connection member 332 is a protrusion (e.g., a projected hub) with sidewalls that extend from the first surface of the base plate 330 and with a first surface substantially parallel to the first surface of the base plate 330. Sidewalls extend from edges of the first surface of the connection member 332 to the first surface of the base plate 330. A remaining portion of the first surface of the base plate 330 surrounding the connection member 332 may be flat or substantially flat. One or more connector features 336 extend outwardly from the sidewalls of the connection member 332 to releasably engage with corresponding connectors of a microneedle enclosure that provides, for example, a sterile environment for the microneedle array 140. The first surface and the sidewalls of the connection member 332 define, in part, a chamber. The chamber may be further defined through a portion of the base plate 330 adjacent (e.g., below) the connection member 332. The chamber has an opening, and is accessible, on the second surface of the base plate 330. An aperture or distal opening 334 is formed through the first surface of the connection member 332. The distal opening 334 may be sized and shaped such that the microneedle array 140 fits securely within and extends through the distal opening 334 when in the deployed configuration. For example, sidewalls of the microneedle array 140 may align with corresponding sidewalls of the distal opening 334. In some variations, the distal opening 334 may be sized and shaped to correspond with an area surrounding the microneedle array 140. The openings in the inner adhesive layer 342 and the outer adhesive layer 344 (or the single adhesive layer) may be sized such that the connection member 332 extends through the openings without interference with the adhesive layers. For example, the diameter of the opening of the inner adhesive layer 342 and the diameter of the opening of the outer adhesive layer 344 is larger than that of the connection member 332. In some variations, the opening of the inner adhesive layer 342 and/or the opening of the outer adhesive layer 344 (or that of the single adhesive layer) is in proximity with the sidewalls of the connection member 332 with a clearance to accommodate the one or more connector features 336. In some variations, one or more slits or notches may be formed in the inner adhesive layer 342, the outer adhesive layer 344, and/or the single adhesive layer, extending from the opening to aid in placement of the respective adhesive layer.

Although the housing cover 320 and the base plate 330 depicted in FIGS. 3A-3D are substantially circular with the housing cover 320 having a dome shape, in other variations, the housing cover 320 and the base plate 330 may have any suitable shape. For example, in other variations the housing cover 320 and the base plate 330 may be generally prismatic and have an elliptical, triangular, rectangular, pentagonal, hexagonal, or other suitable shape. The outer adhesive layer 344 (or the single adhesive layer) may extend outwardly from the housing cover 320 and the base plate 330 to extend beyond the perimeter of the housing cover 320. The outer adhesive layer 344 (or the single adhesive layer) may be circular, as shown in FIGS. 3A-3D or may have an elliptical, triangular, rectangular, pentagonal, hexagonal, or other suitable shape and need not be the same shape as the housing cover 320 and/or the base plate 330.

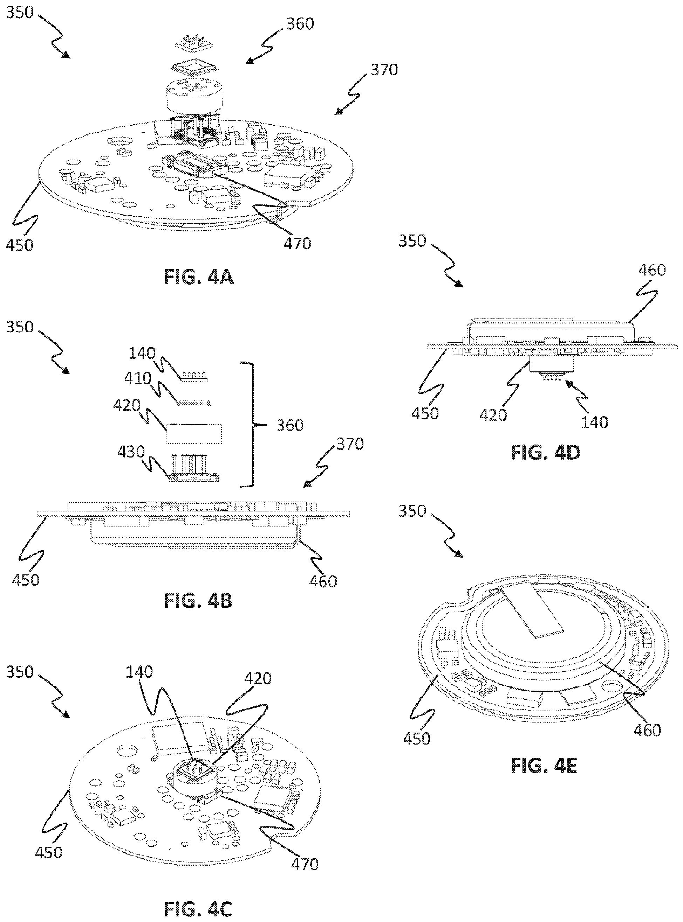

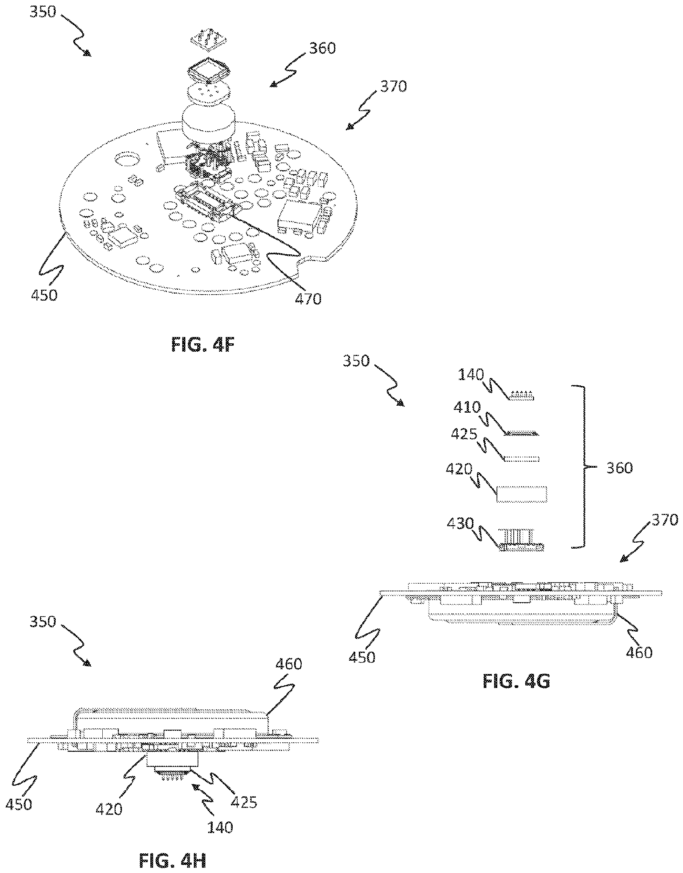

FIGS. 4A-4E depict aspects of the sensor assembly 350 of the analyte monitoring device 110 in a perspective exploded view, a side exploded view, a lower perspective view, a side view, and an upper perspective view, respectively.

The sensor assembly 350 includes microneedle array components and electronic components to implement analyte detection and processing aspects of the microneedle array-based continuous analyte monitoring device 110 for the detection and measuring of an analyte. In some variations, the sensor assembly 350 is a compact, low-profile stack-up that is at least partially contained within the cavity comprising an internal volume defined by the housing cover 320 and the base plate 330.

In some variations, the sensor assembly 350 includes a microneedle array assembly 360 and an electronics assembly 370 that connect to one another to implement the microneedle array analyte detection and processing aspects further described herein. In some variations, the electronics assembly 370 includes a main printed circuit board (PCB) 450 on which electronic components are connected, and the microneedle array assembly 360 includes a secondary printed circuit board (PCB) 420 on which the microneedle array 140 is connected.

In some variations, the microneedle array assembly 360 includes, in addition to the secondary PCB 420 and the microneedle array 140, an epoxy skirt 410 and a secondary PCB connector 430. The microneedle array 140 is coupled to a top side (e.g., outer facing side) of the secondary PCB 420 so that the individual microneedles of the microneedle array 140 are exposed as described with reference to FIG. 3A - FIG. 3D. The secondary PCB connector 430 is coupled to a back side, opposite the top side, of the secondary PCB 420. The secondary PCB connector 430 may be an electromechanical connector and may communicatively couple to the primary PCB 450 through a primary PCB connector 470 on a top side (e.g., outer facing side) of the primary PCB 450 to allow for signal communication between the secondary PCB 420 and the primary PCB 450. For example, signals from the microneedle array 140 may be communicated to the primary PCB 450 through the secondary PCB 420, the secondary PCB connector 430, and the primary PCB connector 470.

The secondary PCB 420 may in part determine the distance to which the microneedle array 140 protrudes from the base plate 330 of the housing. Accordingly, the height of the secondary PCB 420 may be selected to help ensure that the microneedle array 140 is inserted properly into a user's skin. During microneedle insertion, the first surface (e.g., outer facing surface) of the connection member 332 of the base plate 330 may act as a stop for microneedle insertion. If the secondary PCB 420 has a reduced height and its top surface is flush or nearly flush with the first surface of the connection member 332, then the connection member 332 may prevent the microneedle array 140 from being fully inserted into the skin.

In some variations, other components (e.g., electronic components such as sensors or other components) may also be connected to the secondary PCB 420. For example, the secondary PCB 420 may be sized and shaped to accommodate electronic components on the top side or the back side of the secondary PCB 420.

In some variations, the epoxy skirt 410 may be deposited along the edges (e.g., the outer perimeter) of the microneedle array 140 to provide a secure fit of the microneedle array 140 within the distal opening 334 formed in the connection member 332 of the base plate 330 and/or to relieve the sharp edges along the microneedle array 140, as shown in FIG. 3C and FIG. 3D. For example, the epoxy skirt 410 may occupy portions of the distal opening 334 not filled by the microneedle array 140 and/or portions of the chamber defined in the base plate 330 not filled by the secondary PCB 420. The epoxy skirt 410 may also provide a transition from the edges of the microneedle array 140 to the edge of the secondary PCB 420. In some variations, the epoxy skirt 410 may be replaced or supplemented by a gasket (e.g., a rubber gasket) or the like.

The electronics assembly 370, having the primary PCB 450, includes a battery 460 coupled to a back side of the primary PCB 450, opposite the top side on which the primary PCB connector 470 is coupled. In some variations, the battery 460 may be coupled on the top side of the primary PCB 450 and/or in other arrangements.

FIGS. 4F-4H depict aspects of an alternate variation of the sensor assembly 350 of the analyte monitoring device 110. A perspective exploded view, a side exploded view, and a side view of the sensor assembly 350 are provided, respectively, in FIGS. 4F-4H.

As shown, in the sensor assembly 350, an additional PCB component, an intermediate PCB 425, is incorporated. In some variations, the intermediate PCB 425 is part of the microneedle array assembly 360 and is positioned between and connected to the secondary PCB 420 and the microneedle array 140. The intermediate PCB 425 may be added to increase the height of the microneedle array assembly 360 such that the microneedle array 140 extends at a further distance from the base plate 330, which may aid in insertion of the microneedle array 140 into the skin of a user. The microneedle array 140 is coupled to a top side (e.g., outer facing side) of the intermediate PCB 425 so that the individual microneedles of the microneedle array 140 are exposed as described with reference to FIG. 3A - FIG. 3D. The secondary PCB 420 is coupled to a back side, opposite the top side, of the intermediate PCB 425, and the secondary PCB connector 430 is coupled to a back side, opposite the top side, of the secondary PCB 420. The epoxy skirt 410 (which may be replaced or supplemented by a gasket of the like) provides a transition from the edges of the microneedle array 140 to the edge of the intermediate PCB 425.

The intermediate PCB 425 with the secondary PCB 420, in part, determine the distance to which the microneedle array 140 protrudes through the distal opening 334 of the base plate 330. The incorporation of the intermediate PCB 425 provides an additional height to help ensure that the microneedle array 140 is properly inserted into a user's skin. In some variations, the top side (e.g., outer facing side) of the intermediate PCB 425 extends through and out of the distal opening 334 so that the first surface (e.g., top, exposed surface) of the connection member 332 surrounding the distal opening 334 does not prevent the microneedle array from being fully inserted into the skin. In some variations, the top side (e.g., outer facing side) of the intermediate PCB 425 does not extend out of the distal opening 334 but the increased height (by virtue of incorporating the intermediate PCB 425) ensures that the microneedle array 140 protrudes at a sufficient distance from the base plate 330 of the housing.

In some variations, a microneedle enclosure may be provided for releasable attachment to the analyte monitoring device 110. The microneedle enclosure may provide a protective environment or enclosure in which the microneedle array 140 may be safely contained, thereby ensuring the integrity of the microneedle array 140 during certain stages of manufacture and transport of the analyte monitoring device 110, prior to application of the analyte monitoring device 110. The microneedle enclosure is releasable or removable from the analyte monitoring device 110 to allow for the microneedle array 140 to be exposed and/or ready for insertion into the skin of the user, as further described herein.

In some variations, the microneedle enclosure, by providing an enclosed and sealed environment in which the microneedle array 140 may be contained, provides an environment in which the microneedle array 140 may be sterilized. For example, the microneedle enclosure with the microneedle array 140 may be subjected to a sterilization process, during which the sterilization penetrates the microneedle enclosure so that the microneedle array 140 is also sterilized. As the microneedle array 140 is contained in an enclosed environment, the microneedle array 140 remains sterilized until removed from the enclosed environment. In some variations, a removeable film is provided on the distal end of the housing, covering the distal opening 334 prior to application of the analyte monitoring device 110 on the skin surface of a subject. The removeable film may maintain a sterile environment and prevent intrusion of foreign objects or substances before application of the analyte monitoring device 110. A user may remove or peel off the film just prior to applying and/or adhering the analyte monitoring device 110 to the skin surface of a subject.

In some variations, the electronics system of the analyte monitoring device 110 may include an analog front end. The analog front end may include sensor circuitry (e.g., sensor circuitry 124 as shown in FIG. 2A) that converts analog current measurements to digital values that can be processed by the microcontroller. The analog front end may, for example, include a programmable analog front end that is suitable for use with electrochemical sensors. For example, the analog front end may include a MAX30131, MAX30132, or MAX30134 component (which have 1, 2, and 4 channel, respectively), available from Maxim Integrated (San Jose, CA), which are ultra-low power programmable analog front ends for use with electrochemical sensors. The analog front end may also include an AD5940 or AD5941 component, available from Analog Devices (Norwood, MA), which are high precision, impedance and electrochemical front ends. Similarly, the analog front end may also include an LMP91000, available from Texas Instruments (Dallas, TX), which is a configurable analog front end potentiostat for low-power chemical sensing applications. The analog front end may provide biasing and a complete measurement path, including the analog to digital converters (ADCs). Ultra-low power may allow for the continuous biasing of the sensor to maintain accuracy and fast response when measurement is required for an extended duration (e.g. 7 days) using a body-worn, battery-operated device.

In some variations, the analog front end device may be compatible with both two and three terminal electrochemical sensors, such as to enable both DC current measurement, AC current measurement, and electrochemical impedance spectroscopy (EIS) measurement capabilities. Furthermore, the analog front end may include an internal temperature sensor and programmable voltage reference, support external temperature monitoring and an external reference source and integrate voltage monitoring of bias and supply voltages for safety and compliance.

In some variations, the analog front end may include a multi-channel potentiostat to multiplex sensor inputs and handle multiple signal channels. For example, the analog front end may include a multi-channel potentiostat such as that described in U.S. Patent No. 9,933,387, which is incorporated herein in its entirety by this reference.

In some variations, the analog front end and peripheral electronics may be integrated into an application-specific integrated circuit (ASIC), which may help reduce cost, for example. This integrated solution may include the microcontroller described below, in some variations.

In some variations, the electronics system of the analyte monitoring device may include at least one microcontroller (e.g., controller 122 as shown in FIG. 2A). The microcontroller may include, for example, a processor with integrated flash memory. In some variations, the microcontroller in the analyte monitoring device may be configured to perform analysis to correlate sensor signals to an analyte measurement (e.g., glucose measurement). For example, the microcontroller may execute a programmed routine in firmware to interpret the digital signal (e.g., from the analog front end), perform any relevant algorithms and/or other analysis, and route processed data to and/or from the communication module. Keeping the analysis on-board the analyte monitoring device may, for example, enable the analyte monitoring device to broadcast analyte measurement(s) to multiple devices (e.g., mobile computing devices such as a smartphone or smartwatch, therapeutic delivery systems such as insulin pens or pumps, etc.) in parallel, while ensuring that each connected device has the same information.

In some variations, the microcontroller may be configured to activate and/or inactivate the analyte monitoring device on one or more detected conditions. For example, the device may be configured to power on the analyte monitoring device upon deployment or insertion of the microneedle array into skin. This may, for example, enable a power-saving feature in which the battery is disconnected until the microneedle array is deployed, at which time the device may begin broadcasting sensor data. Such a feature may, for example, help improve the shelf life of the analyte monitoring device and/or simplify the analyte monitoring device-external device pairing process for the user.

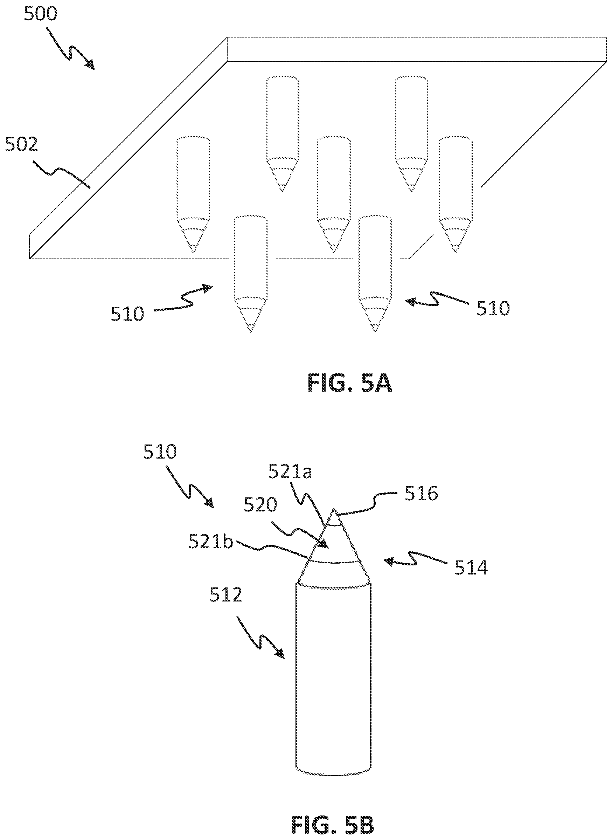

As shown in the schematic of FIG. 5A, in some variations, a microneedle array 510 for use in sensing an analyte may include one or more microneedles 510 projecting from a substrate surface 502. The substrate surface 502 may, for example, be a generally planar semiconductor (e.g. Silicon) substrate and one or more microneedles 510 may project orthogonally from the planar surface. Generally, as shown in FIG. 5B, a microneedle 510 may include a body portion 512 (e.g., shaft) and a tapered distal portion 514 configured to puncture the skin of a user. In some variations, the tapered distal portion 514 may terminate in an insulated distal apex 516. The microneedle 510 may further include an electrode 520 on a surface of the tapered distal portion. In some variations, electrode-based measurements may be performed at the interface of the electrode and interstitial fluid located within the body (e.g., on an outer surface of the overall microneedle). In some variations, the microneedle 510 may have a solid core (e.g., solid body portion), though in some variations the microneedle 510 may include one or more lumens, which may be used for drug delivery or sampling of the dermal interstitial fluid, for example. Other microneedle variations, such as those described below, may similarly either include a solid core or one or more lumens.

The microneedle array 500 may be at least partially formed from a semiconductor (e.g., silicon) substrate and include various material layers applied and shaped using various suitable microelectromechanical systems (MEMS) manufacturing techniques (e.g., deposition and etching techniques), as further described below. The microneedle array may be reflow-soldered to a circuit board, similar to a typical integrated circuit. Furthermore, in some variations the microneedle array 500 may include a three electrode setup including a working (sensing) electrode having an electrochemical sensing coating (including a biorecognition element such as an aptamer or an enzyme) that enables detection of the analyte, a reference electrode, and a counter electrode. In other words, the microneedle array 500 may include at least one microneedle 510 that includes a working electrode, at least one microneedle 510 including a reference electrode, and at least one microneedle 510 including a counter electrode. Additional details of these types of electrodes are described in further detail below.

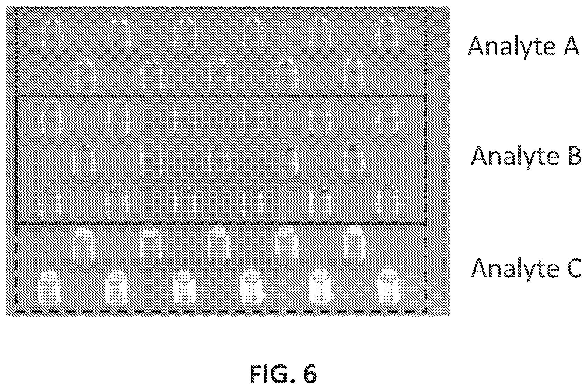

In some variations, the microneedle array 500 may include a plurality of microneedles that are insulated such that the electrode on each microneedle in the plurality of microneedles is individually addressable and electrically isolated from every other electrode on the microneedle array. The resulting individual addressability of the microneedle array 500 may enable greater control over each electrode's function, since each electrode may be separately probed. For example, the microneedle array 500 may be used to provide multiple independent measurements of a given analyte, which improves the device's sensing reliability and accuracy. Furthermore, in some variations the electrodes of multiple microneedles may be electrically connected to produce augmented signal levels. As another example, the same microneedle array 500 may additionally or alternatively be interrogated to simultaneously measure multiple analytes to provide a more comprehensive assessment of physiological status. For example, as shown in the schematic of FIG. 6, a microneedle array may include a portion of microneedles to detect s first analyte A, a second portion of microneedles to detect a second Analyte B, and a third portion of microneedles to detect a third Analyte C. It should be understood that the microneedle array may be configured to detect any suitable number of analytes (e.g., 1, 2, 3, 4, 5 or more, etc.), provided that at least one of the analytes is analyte.

In some variations of microneedles (e.g., microneedles with a working electrode), the electrode 520 may be located proximal to the insulated distal apex 516 of the microneedle. In other words, in some variations the electrode 520 does not cover the apex of the microneedle. Rather, the electrode 520 may be offset from the apex or tip of the microneedle. The electrode 520 being proximal to or offset from the insulated distal apex 516 of the microneedle advantageously provides more accurate sensor measurements. For example, this arrangement prevents concentration of the electric field at the microneedle apex 516 during manufacturing, thereby avoiding non-uniform electro-deposition of sensing chemistry on the electrode surface 520 that would result in faulty sensing. The electrode 520 may be configured to have an annular shape and may comprise a distal edge 521a and a proximal edge 521b.

As another example, placing the electrode 520 offset from the microneedle apex further improves sensing accuracy by reducing undesirable signal artefacts and/or erroneous sensor readings caused by stress upon microneedle insertion. The distal apex of the microneedle is the first region to penetrate into the skin, and thus experiences the most stress caused by the mechanical shear phenomena accompanying the tearing or cutting of the skin. If the electrode 520 were placed on the apex or tip of the microneedle, this mechanical stress may delaminate the electrochemical sensing coating on the electrode surface when the microneedle is inserted, and/or cause a small yet interfering amount of tissue to be transported onto the active sensing portion of the electrode. Thus, placing the electrode 520 sufficiently offset from the microneedle apex may improve sensing accuracy. For example, in some variations, a distal edge 521a of the electrode 520 may be located at least about 10 µm (e.g., between about 20 µm and about 30 µm) from the distal apex or tip of the microneedle, as measured along a longitudinal axis of the microneedle.

The body portion 512 of the microneedle 510 may further include an electrically conductive pathway extending between the electrode 520 and a backside electrode or other electrical contact (e.g., arranged on a backside of the substrate of the microneedle array). The backside electrode may be soldered to a circuit board, enabling electrical communication with the electrode 520 via the conductive pathway. For example, during use, the in-vivo sensing current (inside the dermis) measured at a working electrode is interrogated by the backside electrical contact, and the electrical connection between the backside electrical contact and the working electrode is facilitated by the conductive pathway. In some variations, this conductive pathway may be facilitated by a metal via running through the interior of the microneedle body portion (e.g., shaft) between the microneedle's proximal and distal ends. Alternatively, in some variations the conductive pathway may be provided by the entire body portion being formed of a conductive material (e.g., doped silicon). In some of these variations, the complete substrate on which the microneedle array 500 is built upon may be electrically conductive, and each microneedle 510 in the microneedle array 500 may be electrically isolated from adjacent microneedles 510 as described below. For example, in some variations, each microneedle 510 in the microneedle array 500 may be electrically isolated from adjacent microneedles 510 with an insulative barrier including electrically insulative material (e.g., dielectric material such as silicon dioxide) that surrounds the conductive pathway extending between the electrode 520 and backside electrical contact. For example, body portion 512 may include an insulative material that forms a sheath around the conductive pathway, thereby preventing electrical communication between the conductive pathway and the substrate. Other example variations of structures enabling electrical isolation among microneedles are described in further detail below.

Such electrical isolation among microneedles in the microneedle array permits the sensors to be individually addressable. This individual addressability advantageously enables independent and parallelized measurement among the sensors, as well as dynamic reconfiguration of sensor assignment (e.g., to different analytes). In some variations, the electrodes in the microneedle array can be configured to provide redundant analyte measurements, which is an advantage over conventional analyte monitoring devices. For example, redundancy can improve performance by improving accuracy (e.g., averaging multiple analyte measurement values from different microneedles which reduces the effect of extreme high or low sensor signals on the determination of analyte levels) and/or improving reliability of the device by reducing the likelihood of total failure.

In some variations, as described in further detail below with respective different variations of the microneedle, the microneedle array may be formed at least in part with suitable semiconductor and/or MEMS fabrication techniques and/or mechanical cutting or dicing. Such processes may, for example, be advantageous for enabling large-scale, cost-efficient manufacturing of microneedle arrays.

Described herein are further example variations of microneedle structures incorporating one or more of the above-described microneedle features for a microneedle array in an analyte monitoring device.

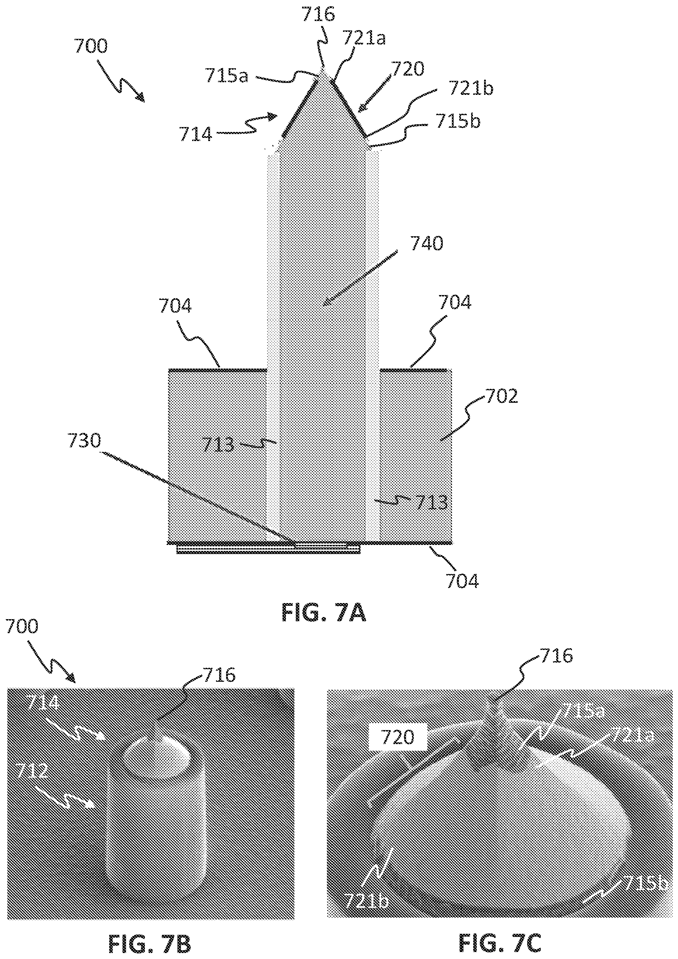

In some variations, a microneedle may have a generally columnar body portion and a tapered distal portion with an electrode. For example, FIGS. 7A-7C illustrate an example variation of a microneedle 700 extending from a substrate 702. FIG. 7A is a side cross-sectional view of a schematic of microneedle 700, while FIG. 7B is a perspective view of the microneedle 700 and FIG. 7C is a detailed perspective view of a distal portion of the microneedle 700. As shown in FIGS. 7B and 7C, the microneedle 700 may include a columnar body portion 712, a tapered distal portion 714 terminating in an insulated distal apex 716, and an annular electrode 720. The annular electrode 720 includes a conductive material (e.g., Pt, Ir, Au, Ti, Cr, Ni, combinations thereof, etc.) arranged on the tapered distal portion 714, such as, for example, on a segment thereof, and comprises a distal edge 721a and a proximal edge 721b. As shown in FIG. 7A, the annular electrode 720 may be proximal to (offset or spaced apart from) the distal apex 716. The annular electrode 720 may be electrically isolated from the distal apex 716 by a distal insulating surface 715a including an insulating material (e.g., SiO2). For example, the distal edge 721a of the annular electrode 720 may be proximate to a proximal edge of the distal insulating surface 715a of the insulated distal apex 716. In some variations, the distal edge 721a of the annular electrode 720 may be proximal to (e.g., just proximal to, adjacent, abutting) a proximal edge of the distal apex 716 (a proximal edge of the distal insulating surface 715a), while in other variations, the distal edge 721a of the annular electrode 720 may be distal to (e.g., just distal to, adjacent) the proximal edge of the insulated distal apex 716 (proximal edge of the distal insulating surface 715a), but may remain proximal to the apex itself. Accordingly, in some variations, the annular electrode 720 may overlie a portion of the distal insulating surface 715a, but may remain proximal to (and offset from) the insulated distal apex itself.

Also as shown in FIG. 7A, the proximal edge 721b of the annular electrode 720 may be distal to, and in some variations, offset or spaced apart from, the columnar body portion 712. In some variations, the proximal edge 721b of the annular electrode 720 may also be electrically isolated from the columnar body portion 712 by a second distal insulating surface 715b comprising an insulating material (e.g., SiO2) at a proximal end or region of the tapered distal portion 714. For example, the proximal edge 721b of the annular electrode 720 may be proximate to a distal edge of the second distal insulating surface 715b. In some variations, the proximal edge 721b of the annular electrode 720 may be proximal to (e.g., just proximal to, adjacent, abutting) a distal edge the second distal insulating surface 715b, while in other variations, the proximal edge 721b of the annular electrode 720 may be distal to (e.g., just distal to, adjacent) the distal edge of the second distal insulating surface 715b, but may remain proximal to the columnar body portion 712. Accordingly, in some variations, the annular electrode 720 may overlie a portion of the second distal insulating surface 715b but may remain proximal to (and offset from) the columnar body portion 712. As shown in FIG. 7A and in some other variations, the annular electrode 720 may be on only a segment of the surface of the tapered distal portion 714, and may or may not extend to the columnar boy portion 712.

The electrode 720 may be in electrical communication with a conductive core 740 (e.g., conductive pathway) passing along the body portion 712 to a backside electrical contact 730 (e.g., made of Ni/Au alloy) or other electrical pad in or on the substrate 702. For example, the body portion 712 may include a conductive core material (e.g., highly doped silicon). As shown in FIG. 7A, in some variations, an insulating moat 713 including an insulating material (e.g., SiO2) may be arranged around (e.g., around the perimeter) of the body portion 712 and extend at least partially through the substrate 702. Accordingly, the insulating moat 713 may, for example, help prevent electrical contact between the conductive core 740 and the surrounding substrate 702. The insulating moat 713 may further extend over the surface of the body portion 712. Upper and/or lower surfaces of the substrate 702 may also include a layer of substrate insulation 704 (e.g., SiO2). Accordingly, the insulation provided by the insulating moat 713 and/or substrate insulation 704 may contribute at least in part to the electrical isolation of the microneedle 700 that enables individual addressability of the microneedle 700 within a microneedle array. Furthermore, in some variations the insulating moat 713 extending over the surface of the body portion 712 may function to increase the mechanical strength of the microneedle 700 structure.

The microneedle 700 may be formed at least in part by suitable MEMS fabrication techniques such as plasma etching, also called dry etching. For example, in some variations, the insulating moat 713 around the body portion 712 of the microneedle may be made by first forming a trench in a silicon substrate by deep reactive ion etching (DRIE) from the backside of the substrate, then filling that trench with a sandwich structure of SiO2 / polycrystalline silicon (poly-Si) / SiO2 by low pressure chemical vapor deposition (LPCVD) or other suitable process. In other words, the insulating moat 713 may passivate the surface of the body portion 712 of the microneedle, and continue as a buried feature in the substrate 702 near the proximal portion of the microneedle. By including largely compounds of silicon, the insulating moat 713 may provide good fill and adhesion to the adjoining silicon walls (e.g., of the conductive core 740, substrate 702, etc.). The sandwich structure of the insulating moat 713 may further help provide excellent matching of coefficient of thermal expansion (CTE) with the adjacent silicon, thereby advantageously reducing faults, cracks, and/or other thermally-induced weaknesses in the insulating structure 713.

The tapered distal portion may be fashioned out by an isotropic dry etch from the frontside of the substrate, and the body portion 712 of the microneedle 700 may be formed from DRIE. The frontside metal electrode 720 may be deposited and patterned on the distal portion by specialized lithography (e.g., electron-beam evaporation) that permits metal deposition in the desired annular region for the electrode 720 without coating the distal apex 716. Furthermore, the backside electrical contact 730 of Ni/Au may be deposited by suitable MEMS manufacturing techniques (e.g., sputtering).

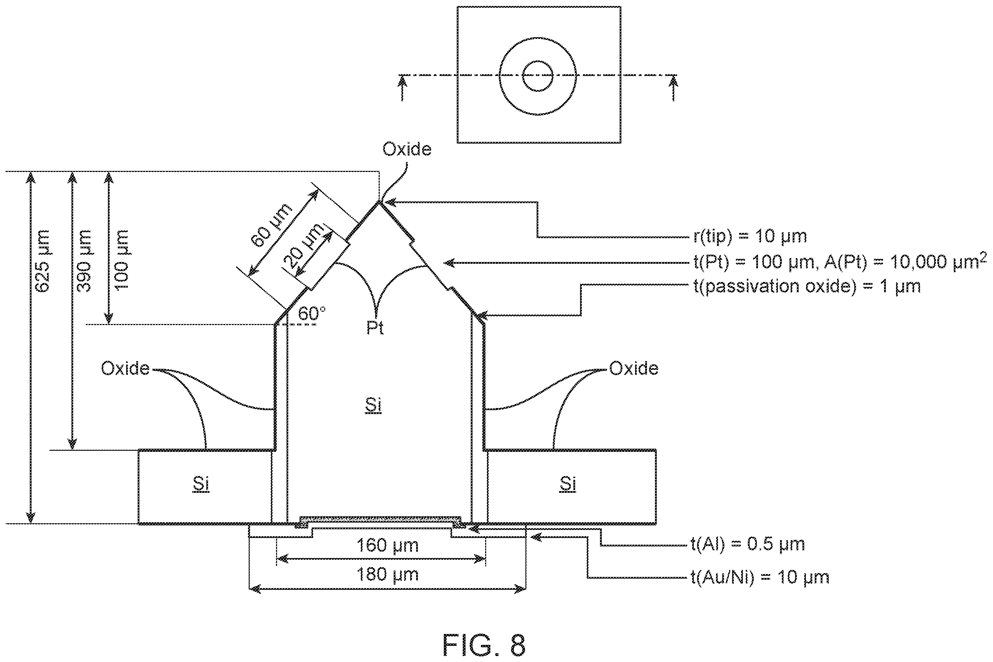

The microneedle 700 may have any suitable dimensions. By way of illustration, the microneedle 700 may, in some variations, have a height of between about 300 µm and about 500 µm. In some variations, the tapered distal portion 714 may have a tip angle between about 60 degrees and about 80 degrees, and an apex diameter of between about 1 µm and about 15 µm. In some variations, the surface area of the annular electrode 720 may include between about 9,000 µm² and about 11,000 µm², or about 10,000 µm². FIG. 8 illustrates various dimensions of an example variation of a columnar microneedle with a tapered distal portion and annular electrode, similar to microneedle 700 described above. As with the microneedle 700 described above, the columnar microneedle of FIG. 8 comprises a columnar body portion, a tapered distal portion terminating in an insulated distal apex, a contact trench formed within the tapered distal portion, and an annular electrode (denoted by "Pt" in FIG. 8) that is arranged on the tapered distal portion and overlays the contact trench. The annular electrode may comprise a conductive material (e.g., Pt, Ir, Au, Ti, Cr, Ni, combinations thereof, etc.). In some variations, the contact trench may have a width of about 1 µm, about 2 µm, about 3 µm, about 4 µm, about 5 µm, about 10 µm, about 15 µm, about 20 µm, about 25 µm, about 30 µm, about 35 µm, about 40 µm, about 45 µm, about 50 µm, or, as shown in FIG. 8, about 20 µm. The annular electrode may comprise a distal edge and a proximal edge, and in some variations, a distance between the distal edge and the proximal edge of the annular electrode may be about 20 µm, about 30 µm, about 40 µm about 50 µm, about 60 µm, about 70 µm, about 80 µm, about 90 µm, about 100 µm, or, as shown in FIG. 8, about 60 µm. In some variations, and as shown in FIG. 8 by the dimensional callouts 60 µm and 20 µm, the annular electrode may overlie the contact trench and, in some instances, a portion of the insulating surfaces (denoted by "Oxide" in FIG. 8) of the tapered distal portion.

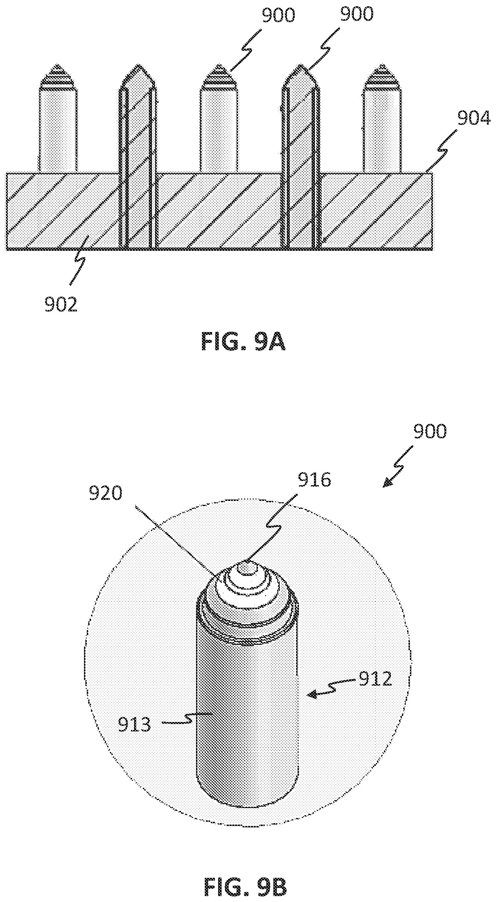

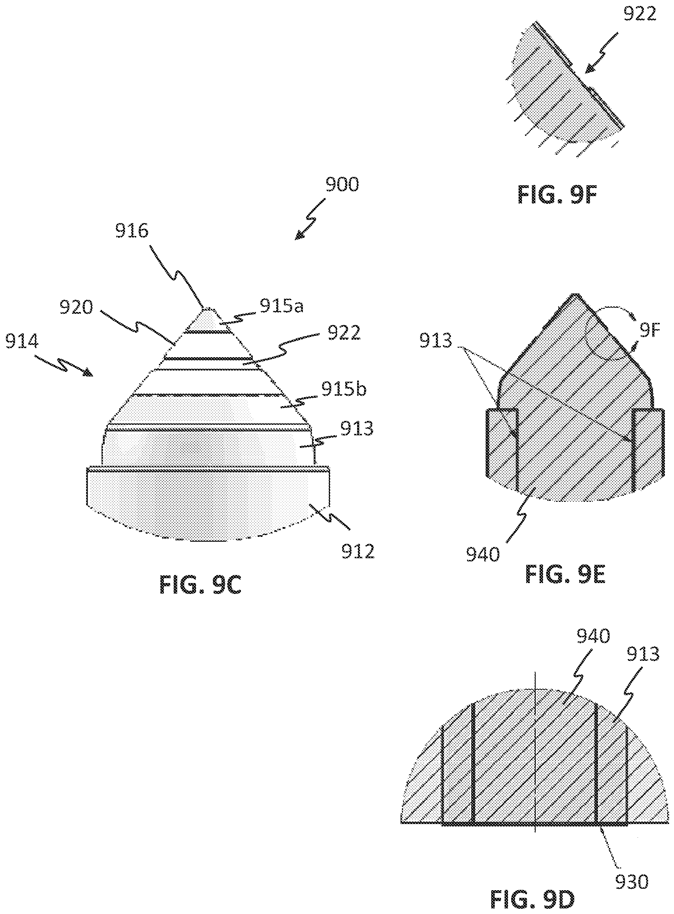

FIGS. 9A-9F illustrate another example variation of a microneedle 900 having a generally columnar body portion extending from a substrate 902 having a top surface 904. The microneedle 900 may be similar to microneedle 700 as described above, except as described below. For example, as shown in FIG. 9B, like the microneedle 700, the microneedle 900 may include a columnar body portion 912, and a tapered distal portion arranged on a cylinder 913 and terminating in an insulated distal apex 916. The cylinder 913 may be insulated and have a smaller diameter than the columnar body portion 912. The microneedle 900 may further include an annular electrode 920 that includes a conductive material and is arranged on the tapered distal portion at a location proximal to (or offset or spaced apart from) the distal apex 916. The electrode 920 may be in electrical communication with a conductive core 940 (e.g., conductive pathway) passing along the body portion 912 to a backside electrical contact 930 (e.g., made of Ni/Au alloy) or other electrical pad in or on the substrate 902. Other elements of microneedle 900 as shown in FIGS. 9A-9F have numbering similar to corresponding elements of microneedle 700.

As can most easily be seen in FIGS. 9B, 9C and 9F, the tapered distal portion 914, and more specifically, the electrode 920 on the tapered distal portion 914 of the microneedle 900, may include a tip contact trench 922. This contact trench may be configured to establish ohmic contact between the electrode 920 and the underlying conductive core 940 of the microneedle. In some variations, the shape of the tip contact trench 922 may include an annular recess formed in the surface of the tapered distal portion 914. In some variations, the shape of the tip contact trench 922 may include an annular recess formed in the surface of the conductive core 940 (e.g., into the body portion of the microneedle, or otherwise in contact with a conductive pathway in the body portion). In some variations, the tip contact trench 922 may be formed in the insulating material on the tapered distal portion 914, and may have a depth about equal to the thickness of the insulating material (e.g., the distal insulating surface 915a and/or the second distal insulating surface 915b). In some instances, the depth of the contact trench may be greater than the thickness of the insulating material such that the contact trench extends beyond a surface of the conductive core 940 (e.g., into the conductive core 940). The electrode 920 may overlie the tip contact trench 922 such that ohmic contact is established between the electrode 920 and the conductive core 940. In some variations, the electrode 920 may extend beyond the tip contact trench 922 such that when the electrode 920 material is deposited onto the conductive core 940, the electrode 920 with the tip contact trench 922 may have a stepped profile when viewed from the side. The tip contact trench 922 may thus advantageously help ensure contact between the electrode 920 and the underlying conductive core 940. Any of the other microneedle variations described herein may also have a similar tip contact trench to help ensure contact between the electrode (which may be, for example, a working electrode, reference electrode, counter electrode, etc.) with a conductive pathway within the microneedle.

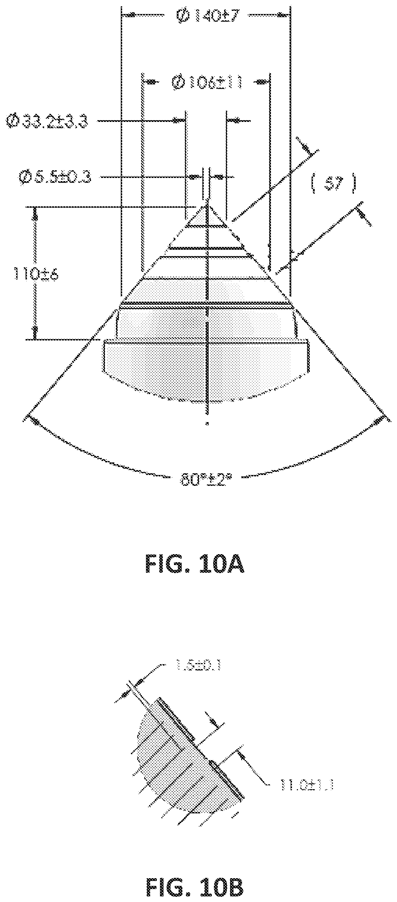

FIGS. 10A and 10B illustrate additional various dimensions of an example variation of a columnar microneedle with a tapered distal portion and annular electrode, similar to microneedle 900 described above. For example, the variation of the microneedle shown in FIGS. 10A and 10B may have a tapered distal portion generally having a taper angle of about 80 degrees (or between about 78 degrees and about 82 degrees, or between about 75 degrees and about 85 degrees), and a cone diameter of about 140 µm (or between about 133 µm and about 147 µm, or between about 130 µm and about 150 µm). The cone of the tapered distal portion may be arranged on a cylinder such that the overall combined height of the cone and cylinder is about 110 µm (or between about 99 µm and about 116 µm, or between about 95 µm and about 120 µm). The annular electrode on the tapered distal portion may have an outer or base diameter of about 106 µm (or between about 95 µm and about 117 µm, or between about 90 µm and about 120 µm), and an inner diameter of about 33.2 µm (or between about 30 µm and about 36 µm, or between about 25 µm and about 40 µm). The length of the annular electrode, as measured along the slope of the tapered distal portion, may be about 57 µm (or between about 55 µm and about 65 µm), and the overall surface area of the electrode may be about 12,700 µm² (or between about 12,500 µm² and about 12,900 µm², or between about 12,000 µm² and about 13,000 µm²). As shown in FIG. 10B, the electrode may furthermore have a tip contact trench extending around a central region of the cone of the tapered distal portion, where the contact may have a width of about 11 µm (or between about 5 µm and about 50 µm , between about 10 µm and about 12 µm, or between about 8 µm and about 14 µm) as measured along the slope of the tapered distal portion, and a trench depth of about 1.5 µm (or between about 0.1 µm and about 5 µm , or between about 0.5 µm and about 1.5 µm , or between about 1.4 µm and about 1.6 µm, or between about 1 µm and about 2 µm). The microneedle has an insulated distal apex having a diameter of about 5.5 µm (or between about 5.3 µm and about 5.8 µm, or between about 5 µm and about 6 µm).

Details of example variations of microneedle array configurations are described in further detail below.

As described above, each microneedle in the microneedle array may include an electrode. In some variations, multiple distinct types of electrodes may be included among the microneedles in the microneedle array. For example, in some variations the microneedle array may function as an electrochemical cell operable in an electrolytic manner with three types of electrodes. In other words, the microneedle array may include at least one working electrode, at least one counter electrode, and at least one reference electrode. Thus, the microneedle array may include three distinct electrode types, though one or more of each electrode type may form a complete system (e.g., the system might include multiple distinct working electrodes). Furthermore, multiple distinct microneedles may be electrically joined to form an effective electrode type (e.g., a single working electrode may be formed from two or more connected microneedles with working electrode sites). Each of these electrode types may include a metallization layer and may include one or more coatings or layers over the metallization layer that help facilitate the function of that electrode.

Generally, the working electrode is the electrode at which oxidation and/or reduction reaction of interest occurs for detection of an analyte of interest. The counter electrode functions to source (provide) or sink (accumulate) the electrons, via an electrical current, that are required to sustain the electrochemical reaction at the working electrode. The reference electrode functions to provide a reference potential for the system; that is, the electrical potential at which the working electrode is biased is referenced to the reference electrode. A fixed, time-varying, or at least controlled potential relationship is established between the working and reference electrodes, and within practical limits no current is sourced from or sinked to the reference electrode. Additionally, to implement such a three-electrode system, the analyte monitoring device may include a suitable potentiostat or electrochemical analog front end to maintain a fixed potential relationship between the working electrode and reference electrode contingents within the electrochemical system (via an electronic feedback mechanism), while permitting the counter electrode to dynamically swing to potentials required to sustain the redox reaction of interest.

Multiple microneedles (e.g., any of the microneedle variations described herein, each of which may have a working electrode, counter electrode, or reference electrode as described above) may be arranged in a microneedle array. Considerations of how to configure the microneedles include factors such as desired insertion force for penetrating skin with the microneedle array, optimization of electrode signal levels and other performance aspects, manufacturing costs and complexity, etc.

For example, the microneedle array may include multiple microneedles that are spaced apart at a predefined pitch (distance between the center of one microneedle to the center of its nearest neighboring microneedle). In some variations, the microneedles may be spaced apart with a sufficient pitch so as to distribute force (e.g., avoid a "bed of nails" effect) that is applied to the skin of the user to cause the microneedle array to penetrate the skin. As pitch increases, force required to insert the microneedle array tends to decrease and depth of penetration tends to increase. However, it has been found that pitch only begins to affect insertion force at low values (e.g., less than about 150 µm). Accordingly, in some variations the microneedles in a microneedle array may have a pitch of at least 200 µm, at least 300 µm, at least 400 µm, at least 500 µm, at least 600 µm, at least 700 µm, or at least 750 µm. For example, the pitch may be between about 200 µm and about 800 µm, between about 300 µm and about 700 µm, or between about 400 µm and about 600 µm. In some variations, the microneedles may be arranged in a periodic grid, and the pitch may be uniform in all directions and across all regions of the microneedle array. Alternatively, the pitch may be different as measured along different axes (e.g., X, Y directions) and/or some regions of the microneedle array may include a smaller pitch while others may include a larger pitch.

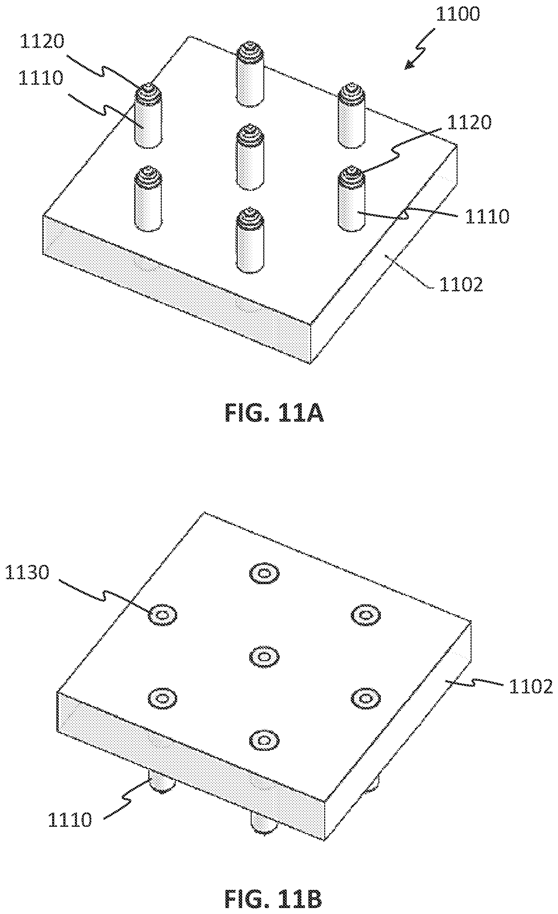

Furthermore, for more consistent penetration, microneedles may be spaced equidistant from one another (e.g., same pitch in all directions). To that end, in some variations, the microneedles in a microneedle array may be arranged in a hexagonal configuration as shown in FIGS. 11A-11C, 12A-12B, and 13A-13E. Alternatively, the microneedles in a microneedle array may arranged in a rectangular array (e.g., square array), or in another suitable symmetrical manner

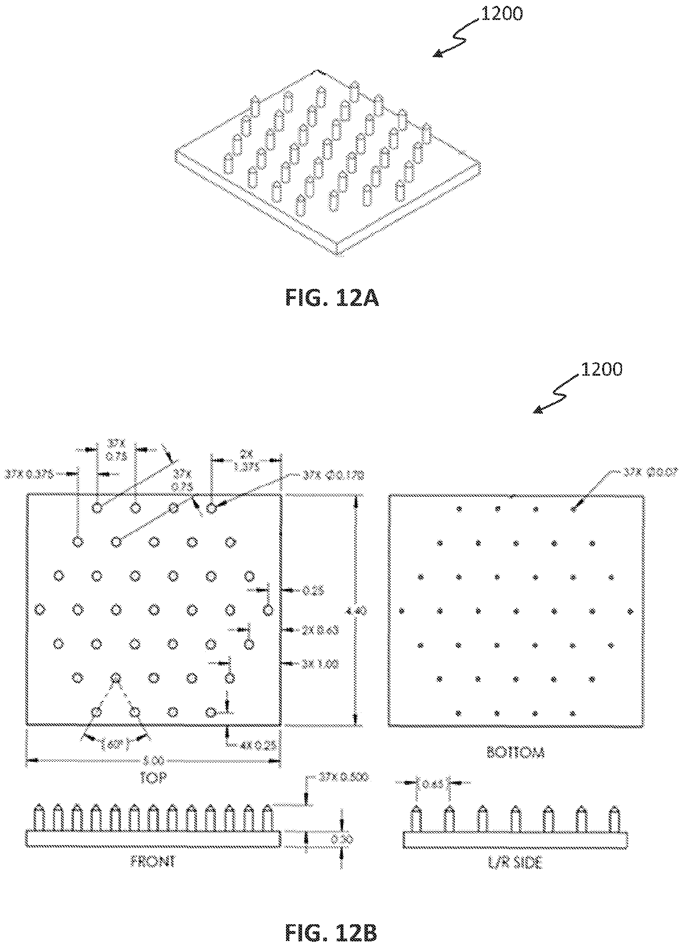

Another consideration for determining configuration of a microneedle array is overall signal level provided by the microneedles. Generally, signal level at each microneedle is invariant of the total number of microneedle elements in an array. However, signal levels can be enhanced by electrically interconnecting multiple microneedles together in an array. For example, an array with a large number of electrically connected microneedles is expected to produce a greater signal intensity (and hence increased accuracy) than one with fewer microneedles. However, a higher number of microneedles on a die will increase die cost (given a constant pitch) and will also require greater force and/or velocity to insert into skin. In contrast, a lower number of microneedles on a die may reduce die cost and enable insertion into the skin with reduced application force and/or velocity. Furthermore, in some variations a lower number of microneedles on a die may reduce the overall footprint area of the die, which may lead to less unwanted localized edema and/or erythema. Accordingly, in some variations, a balance among these factors may be achieved with a microneedle array including 37 microneedles as shown in FIGS. 12A-12B or a microneedle array including seven microneedles as shown in FIGS. 11A-11C. However, in other variations there may be fewer microneedles in an array (e.g., between about 5 and about 35, between about 5 and about 30, between about 5 and about 25, between about 5 and about 20, between about 5 and about 15, between about 5 and about 100, between about 10 and about 30, between about 15 and about 25, etc.) or more microneedles in an array (e.g., more than 37, more than 40, more than 45, etc.).

Additionally, as described in further detail below, in some variations only a subset of the microneedles in a microneedle array may be active during operation of the analyte monitoring device. For example, a portion of the microneedles in a microneedle array may be inactive (e.g., no signals read from electrodes of inactive microneedles). In some variations, a portion of the microneedles in a microneedle array may be activated at a certain time during operation and remain active for the remainder of the operating lifetime of the device. Furthermore, in some variations, a portion of the microneedles in a microneedle array may additionally or alternatively be deactivated at a certain time during operation and remain inactive for the remainder of the operating lifetime of the device.

In considering characteristics of a die for a microneedle array, die size is a function of the number of microneedles in the microneedle array and the pitch of the microneedles. Manufacturing costs are also a consideration, as a smaller die size will contribute to lower cost since the number of dies that can be formed from a single wafer of a given area will increase. Furthermore, a smaller die size will also be less susceptible to brittle fracture due to the relative fragility of the substrate.

Furthermore, in some variations, microneedles at the periphery of the microneedle array (e.g., near the edge or boundary of the die, near the edge or boundary of the housing, near the edge or boundary of an adhesive layer on the housing, along the outer border of the microneedle array, etc.) may be found to have better performance (e.g., sensitivity) due to better penetration compared to microneedles in the center of the microneedle array or die. Accordingly, in some variations, working electrodes may be arranged largely or entirely on microneedles located at the periphery of the microneedle array, to obtain more accurate and/or precise analyte measurements.

FIGS. 12A and 12B depict an illustrative schematic of 37 microneedles arranged in an example variation of a microneedle array 1200. The 37 microneedles may, for example, be arranged in a hexagonal array with an inter-needle center-to-center pitch of about 750 µm (or between about 700 µm and about 800 µm, or between about 725 µm and about 775 µm) between the center of each microneedle and the center of its immediate neighbor in any direction. FIG. 12A depicts an illustrative schematic of an example variation of a die including the microneedle arrangement. Example dimensions of the die (e.g., about 4.4 mm by about 5.0 mm) and the microneedle array 1200 are shown in FIG. 12B.

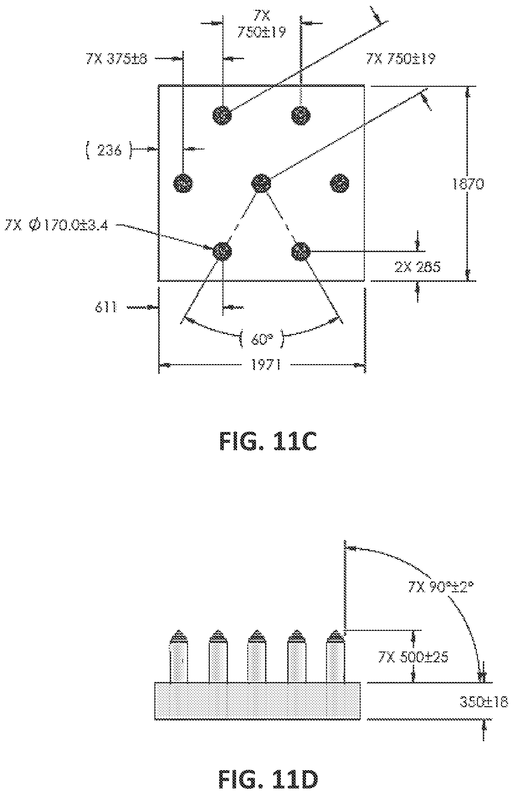

FIGS. 11A and 11B depict perspective views of an illustrative schematic of seven microneedles 1110 arranged in an example variation of a microneedle array 1100. The seven microneedles 1110 are arranged in a hexagonal array on a substrate 1102. As shown in FIG. 11A, the electrodes 1120 are arranged on distal portions of the microneedles 1110 extending from a first surface of the substrate 1102. As shown in FIG. 11B, proximal portions of the microneedles 1110 are conductively connected to respective backside electrical contacts 1130 on a second surface of the substrate 1102 opposite the first surface of the substrate 1102. FIGS. 11C and 11D depict plan and side views of an illustrative schematic of a microneedle array similar to microneedle array 1100. As shown in FIGS. 11C and 11D, the seven microneedles are arranged in a hexagonal array with an inter-needle center-to-center pitch of about 750 µm between the center of each microneedle and the center of its immediate neighbor in any direction. In other variations the inter-needle center-to-center pitch may be, for example, between about 700 µm and about 800 µm, or between about 725 µm and about 775 µm. The microneedles may have an approximate outer shaft diameter of about 170 µm (or between about 150 µm and about 190 µm, or between about 125 µm and about 200 µm) and a height of about 500 µm (or between about 475 µm and about 525 µm, or between about 450 µm and about 550 µm).

Furthermore, the microneedle arrays described herein may have a high degree of configurability concerning where the working electrode(s), counter electrode(s), and reference electrode(s) are located within the microneedle array. This configurability may be facilitated by the electronics system.

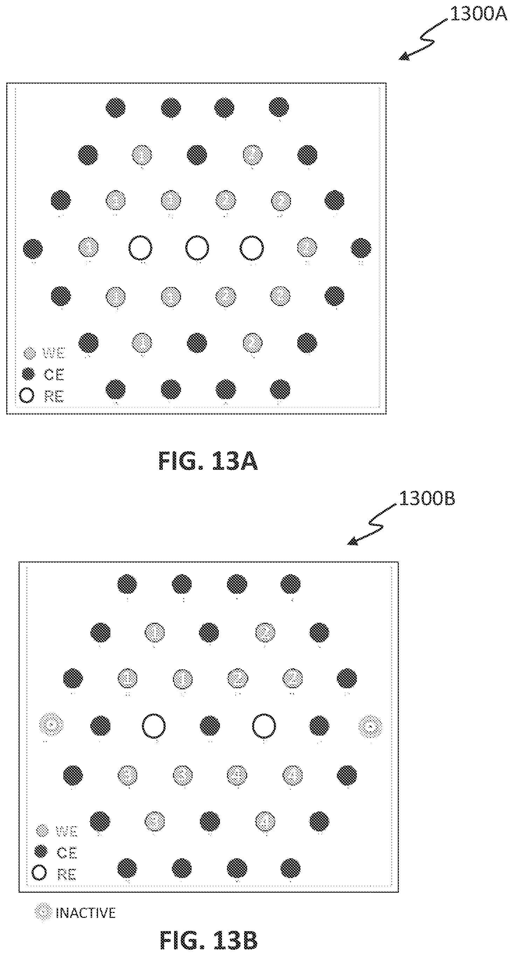

In some variations, a microneedle array may include electrodes distributed in two or more groups in a symmetrical or non-symmetrical manner in the microneedle array, with each group featuring the same or differing number of electrode constituents depending on requirements for signal sensitivity and/or redundancy. For example, electrodes of the same type (e.g., working electrodes) may be distributed in a bilaterally or radially symmetrical manner in the microneedle array. For example, FIG. 13A depicts a variation of a microneedle array 1300A including two symmetrical groups of seven working electrodes (WE), with the two working electrode groups labeled "1" and "2". In this variation, the two working electrode groups are distributed in a bilaterally symmetrical manner within the microneedle array. The working electrodes are generally arranged between a central region of three reference electrodes (RE) and an outer perimeter region of twenty counter electrodes (CE). In some variations, each of the two working electrode groups may include seven working electrodes that are electrically connected amongst themselves (e.g., to enhance sensor signal). Alternatively, only a portion of one or both of the working electrode groups may include multiple electrodes that are electrically connected amongst themselves. As yet another alternative, the working electrode groups may include working electrodes that are standalone and not electrically connected to other working electrodes. Furthermore, in some variations the working electrode groups may be distributed in the microneedle array in a non-symmetrical or random configuration.

As another example, FIG. 13B depicts a variation of a microneedle array 1300B including four symmetrical groups of three working electrodes (WE), with the four working electrode groups labeled "1", "2", "3", and "4." In this variation, the four working electrode groups are distributed in a radially symmetrical manner in the microneedle array. Each working electrode group is adjacent to one of two reference electrode (RE) constituents in the microneedle array and arranged in a symmetrical manner. The microneedle array also includes counter electrodes (CE) arranged around the perimeter of the microneedle array, except for two electrodes on vertices of the hexagon that are inactive or may be used for other features or modes of operation.

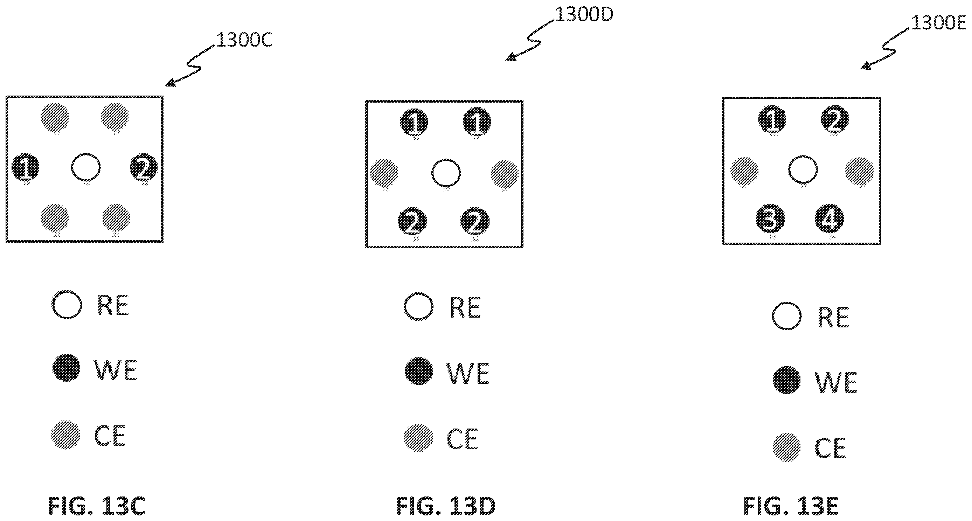

FIG. 13C depicts another example variation of a microneedle array 1300C with seven microneedles. The microneedle arrangement contains two microneedles assigned as independent working electrodes (1 and 2), a counter electrode contingent comprised of 4 microneedles, and a single reference electrode. There is bilateral symmetry in the arrangement of working and counter electrodes, which are equidistant from the central reference electrode. Additionally, the working electrodes are arranged as far as possible from the center of the microneedle array (e.g., at the periphery of the die or array) to take advantage of a location where the working electrodes are expected to have greater sensitivity and overall performance.

FIG. 13D depicts another example variation of a microneedle array 1300D with seven microneedles. The microneedle arrangement contains four microneedles assigned as two independent groupings (1 and 2) of two working electrodes each, a counter electrode contingent comprised of 2 microneedles, and a single reference electrode. There is bilateral symmetry in the arrangement of working and counter electrodes, which are equidistant from the central reference electrode. Additionally, the working electrodes are arranged as far as possible from the center of the microneedle array (e.g., at the periphery of the die or array) to take advantage of a location where the working electrodes are expected to have greater sensitivity and overall performance.

FIG. 13E depicts another example variation of a microneedle array 1300E with seven microneedles. The microneedle arrangement contains four microneedles assigned as independent working electrodes (1, 2, 3, and 4), a counter electrode contingent comprised of 2 microneedles, and a single reference electrode. There is bilateral symmetry in the arrangement of working and counter electrodes, which are equidistant from the central reference electrode. Additionally, the working electrodes are arranged as far as possible from the center of the microneedle array (e.g., at the periphery of the die or array) to take advantage of a location where the working electrodes are expected to have greater sensitivity and overall performance.

While FIGS. 13A-13E illustrate example variations of microneedle array configurations, it should be understood that these figures are not limiting and other microneedle configurations (including different numbers and/or distributions of working electrodes, counter electrodes, and reference electrodes, and different numbers and/or distributions of active electrodes and inactive electrodes, etc.) may be suitable in other variations of microneedle arrays.

As described above, the analyte monitoring device (or various aspects thereof as described above) may be integrated with an applicator or application components configured to urge the microneedle array 140 toward the skin of the user such that the microneedle array 140 is inserted into the skin (e.g., to the desired target depth). In some variations, one or more adhesive layers are provided on a distal end of the housing of the analyte monitoring device and are adhered to the skin to securely hold the analyte monitoring device 110 in place during or prior to deployment of the microneedle array 140 into the skin.

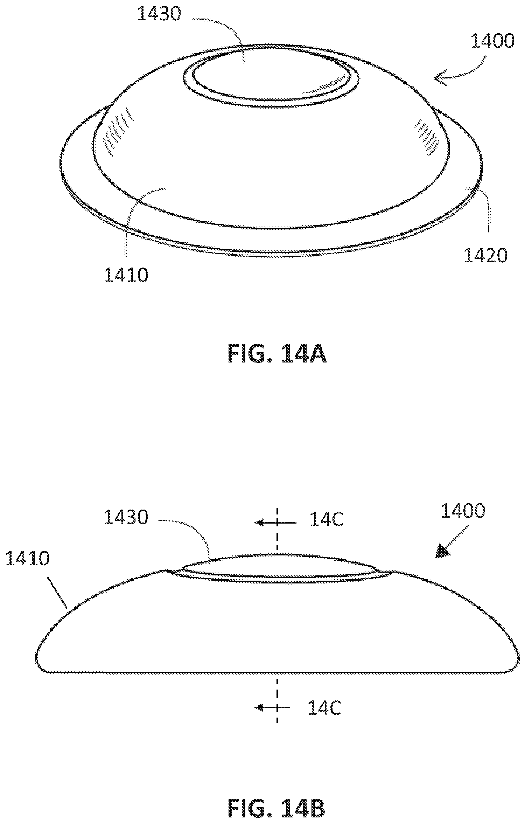

FIG. 14A and FIG. 14B illustrate aspects of a wearable analyte monitoring device with integrated applicator 1400 (also referred to herein as an analyte monitoring device). FIG. 14A provides an upper perspective view and FIG. 14B provides a side view of the analyte monitoring device with integrated applicator 1400. In some variations, the analyte monitoring device 1400 includes a housing cover 1410 and a housing base 1420 that together form a body of a housing and define an internal cavity. An adhesive layer may be provided on a distal, outer-facing region of the housing base 1420 or distal end of the housing to adhere the analyte monitoring device 1400 to the skin of the user.

In some variations, an actuation member 1430 is formed at a proximal surface of the housing cover 1410. The actuation member 1430 is a depressible or releasable (e.g., flexible) member that responds to user force. For example, when the user pushes downward on the actuation member 1430, the actuation member responds by depressing inward. After removal of the user force, the actuation member 1430 may assume its original shape. In some variations, the actuation member 1430 may be a deformable portion of the housing cover 1410. For example, the actuation member 1430 may be made of a material that responds to force and/or pressure. Surrounding portions of the housing cover 1410, in some variations, may be made of a stronger, more resilient material that maintains its shape and structure as the actuation member 1430 deforms upon a force applied by a user. In some variations, the actuation member 1430 may be a component separate from, but coupled with, the housing cover 1410. For example, the actuation member 1430 may be a releasable member such as a cap or button that is fitted within or mated with the surrounding portions of the housing cover 1410. In some variations, the actuation member 1430 may be a diaphragm.

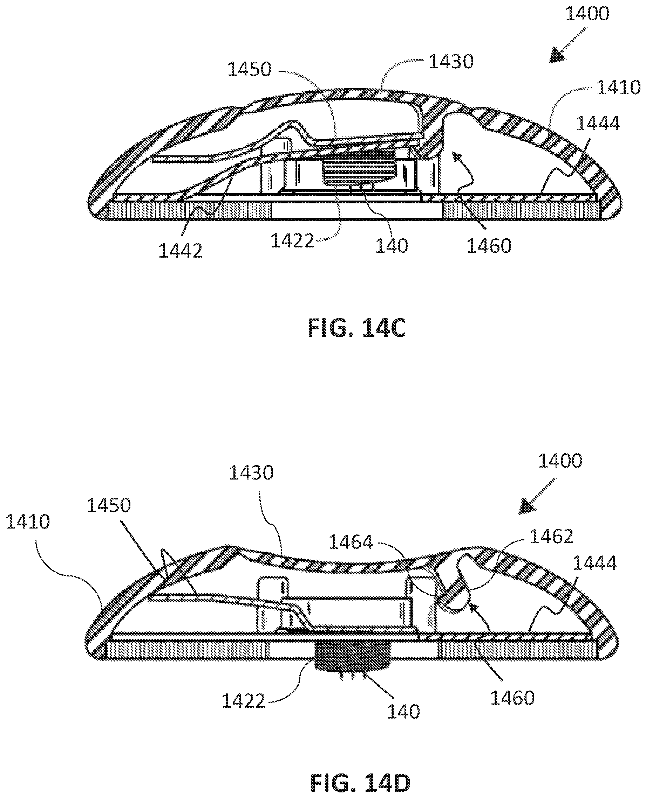

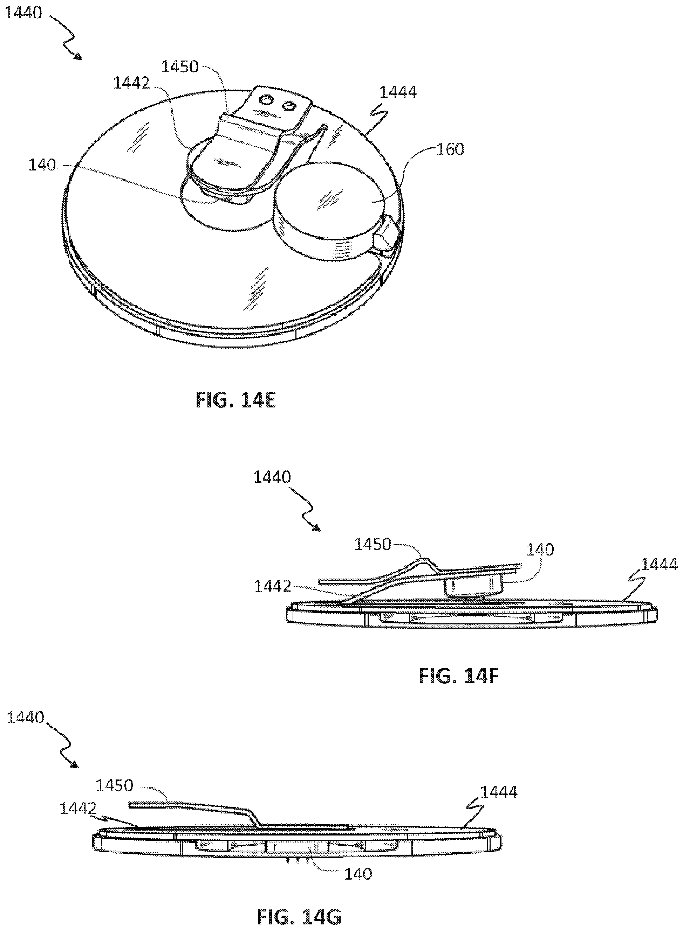

FIG. 14C and FIG. 14D illustrate internal aspects of the analyte monitoring device 1400. FIG. 14C is a side cross-sectional view, taken along the line 14C:14C shown in FIG. 14A, of the analyte monitoring device 1400 in a configuration for deploying the microneedle array 140 of the analyte monitoring device 1400. In FIG. 14C, the microneedle array 140 is in a first configuration in which the microneedle array 140 is held within the cavity of the housing body. FIG. 14D is a side cross-sectional view, taken along the line 14C:14C shown in FIG. 14A, of the analyte monitoring device 1400 in a configuration in which the microneedle array 140 is deployed. In FIG. 14,D the microneedle array 140 is in a second configuration in which the microneedle array 140 protrudes through a distal opening of the housing body.

In some variations, a printed circuit board assembly 1440, including a first assembly portion 1442 and a second assembly portion 1444, is arranged in the housing (e.g., in the cavity defined by the housing cover 1410 and the housing base 1420). The first assembly portion 1442 may be configured to connect to the microneedle array 140. That is, the microneedle array 140 may be electrically connected to the first assembly portion 1442 through, for example, a connection component 1422. The connection component 1422 may be analogous or similar to the secondary PCB component and/or secondary PCB connector described above (e.g., secondary PCB 420 and secondary PCB connector 430 depicted in FIG. 4B and FIG. 4G), such that the connection component 1422 provides an electrical connection between the electrical contacts on the backside of the microneedle array 140 and the first assembly portion 1442 of the printed circuit board assembly 1440.

In some variations, the microneedle array 140 is provided as part of a microneedle array assembly, similar to the microneedle array assembly described above (e.g., microneedle array assembly 360 depicted in FIG. 4B and FIG. 4G). Additionally, a microneedle array assembly utilized in the analyte monitoring device with integrated applicator may comprise a skirt (similar to skirt 410 depicted in FIG. 4B and FIG. 4G) and a spacer or intermediate PCB (similar to intermediate PCB 425 depicted in FIG. 4G).

The second assembly portion 1444 generally surrounds the first assembly portion 1442 and includes other components of the analyte monitoring device as described elsewhere herein (e.g., the electronic components for processing and communicating analyte signals). In some variations, the first assembly portion 1442 comprises a flexible PCB which provides an electrical connection between the microneedle array 140 and the second assembly portion 1444, thereby providing the microneedle array in electrical communication with the other components of the analyte monitoring device. In some variations, the first assembly portion 1442 comprises an elastic material and may be utilized as a biasing element without the need for an additional component. For example, the printed circuit board assembly 1440 may comprise an elastic substrate (e.g., a fiberglass reinforced PCB) which allows for the first assembly portion 1442 to be cut out and utilized as a biasing element while remaining integral with the second assembly portion 1444.

FIG. 14E, FIG. 14F, and FIG. 14G illustrate aspects of the printed circuit board assembly 1440. FIG. 14E provides an upper perspective view, FIG. 14F a side cross-sectional view in a configuration for deploying the microneedle array 140, and FIG. 14G a side cross-sectional view in a configuration for deploying the microneedle array 140. The first assembly portion 1442 may be a flexible circuit board, the flexibility allowing for the movement of the first assembly portion 1442 relative to the second assembly portion 1444. In some variations, a battery 160 is coupled to the second assembly portion 1444. The battery 160 may be offset from the center of the device/second assembly 1444 to allow space for translation of the microneedle array and biasing elements during transition of the microneedle array 140 from the first configuration to the second configuration during the deployment of the microneedle array 140.

As shown in FIGS. 14C-14G, a biasing element 1450 is arranged in the cavity of the housing body of the analyte monitoring device 1400. The biasing element 1450 is attached or otherwise connected to the first assembly portion 1442 of the printed circuit board assembly 1440 containing the microneedle array 140. The biasing element 1450 thus acts as a support structure for the microneedle array 140. The biasing element 1450, which may be a movable clip, a leaf spring, a coiled compression spring, a tension spring, or the like, is positioned in a loaded or first configuration upon assembly of the analyte monitoring device 1400, as shown in FIG. 14C. In this position, the microneedle array 140 is retracted inside the cavity defined by the housing cover 1410 and the housing base 1420 and held in place by an engagement of the biasing element 1450 and a retention element 1460.

The biasing element 1450 may be disengaged from the retention element 1460 upon actuation of the actuation member 1430. For example, by applying force or pressure to an outer surface of the actuation member 1430, the retention element 1460 is released from the biasing element 1450. The release or disengagement of the biasing element 1450 and the retention element 1460 causes an accelerating force on the microneedle array 140, causing insertion into the skin surface of the user. The biasing element 1450 moves from the first, loaded configuration to the second, deployed configuration in which the first biasing element 1450 is compressed into a stressed state and thus presses on the microneedle array 140 with a force (e.g., between about 15 to about 35 Newtons). Once the biasing element 1450 is released via actuation by the user, the first biasing element 1450 applies an accelerating force on the microneedle array 140 in the direction of application.

The biasing element, when loaded, is compressed and/or bent into a stressed state and thus provides potential energy when the microneedle array is in a first configuration. Once the biasing element is released from the retention element via actuation by the user, the biasing element applies an accelerating force on the microneedle array in the direction of application. Because the biasing element is only acting on the microneedle array, and not the entire monitoring device, the force accelerates the microneedle array to relatively high speeds in a very short displacement distance to impact the skin.

In some variations, the biasing element accelerates the microneedle array to a velocity of about 7 to about 14 meters per second (m/s) prior to penetration of the skin surface of the user. In some variations, the biasing element accelerates the microneedle array to a velocity of about 2.5 m/s to about 5 m/s, of about 2.5 m/s to about 7 m/s, of about 2.5 m/s to about 10 m/s, of about 2.5 m/s to about 12.5 m/s, of about 2.5 m/s to about 15 m/s, of about 2.5 m/s to about 20 m/s, of about 2.5 m/s to about 25 m/s, of about 5 m/s to about 7 m/s, of about 5 m/s to about 10 m/s, of about 5 m/s to about 12.5 m/s, of about 5 m/s to about 15 m/s, of about 5 m/s to about 20 m/s, of about 5 m/s to about 25 m/s, of about 7 m/s to about 10 m/s, of about 7 m/s to about 12.5 m/s, of about 7 m/s to about 15 m/s, of about 7 m/s to about 20 m/s, of about 7 m/s to about 25 m/s, of about 10 m/s to about 12.5 m/s, of about 10 m/s to about 15 m/s, of about 10 m/s to about 20 m/s, of about 10 m/s to about 25 m/s, of about 12.5 m/s to about 15 m/s, of about 12.5 m/s to about 20 m/s, of about 12.5 m/s to about 25 m/s, of about 15 m/s to about 20 m/s, of about 15 m/s to about 25 m/s, or of about 20 m/s to about 25 m/s. In some variations, the biasing element accelerates the microneedle array to a velocity of at least about 2.5 m/s, about 5 m/s, about 7 m/s, about 10 m/s, about 12.5 m/s, about 15 m/s, about 20 m/s, or about 25 m/s.

In some variations, the microneedle array is translated about 1.5 to about 3 millimeters (mm) as it is deployed from the first configuration to the second configuration. In some variations, the microneedle array is translated about 0.5 mm to about 1 mm, about 0.5 mm to about 1.5 mm, about 0.5 mm to about 2 mm, about 0.5 mm to about 2.5 mm, about 0.5 mm to about 3 mm, about 0.5 mm to about 5 mm, about 0.5 mm to about 7 mm, about 0.5 mm to about 10 mm, about 1 mm to about 1.5 mm, about 1 mm to about 2 mm, about 1 mm to about 2.5 mm, about 1 mm to about 3 mm, about 1 mm to about 5 mm, about 1 mm to about 7 mm, about 1 mm to about 10 mm, about 1.5 mm to about 2 mm, about 1.5 mm to about 2.5 mm, about 1.5 mm to about 3 mm, about 1.5 mm to about 5 mm, about 1.5 mm to about 7 mm, about 1.5 mm to about 10 mm, about 2 mm to about 2.5 mm, about 2 mm to about 3 mm, about 2 mm to about 5 mm, about 2 mm to about 7 mm, about 2 mm to about 10 mm, about 2.5 mm to about 3 mm, about 2.5 mm to about 5 mm, about 2.5 mm to about 7 mm, about 2.5 mm to about 10 mm, about 3 mm to about 5 mm, about 3 mm to about 7 mm, about 3 mm to about 10 mm, about 5 mm to about 7 mm, about 5 mm to about 10 mm, or about 7 mm to about 10 mm. In some variations, the microneedle array is translated at most about 0.5 mm, 1 mm, 1.5 mm, 2 mm, 2.5 mm, 3 mm, 5 mm, 7 mm, or 10 mm.

In variations, the retention element 1460 is integral with the actuation member 1430 and/or coupled thereto. The retention element 1460 may include an extension arm 1462 with a retention ledge 1464. The retention ledge 1464 provides a support surface for the first biasing element 1450. An outer edge of the biasing element 1450 rests on, interfaces with, and/or is otherwise engaged with the retention ledge 1464 when loaded.

In response to actuation of the actuation member 1430 (e.g., pressure or force applied by the user), the biasing element 1450 and the retention element 1460 disengage. The retention element 1460 may flex and/or move in a downward direction in response to the actuation, allowing for the disengagement between the biasing element 1450 and the retention element 1460. In some variations, as depicted in FIG. 14D, the actuation member 1430 is integrated with the housing cover 1430. The actuation member 1430 may be provided as a flexible portion of the housing cover 1410 which becomes inverted upon depression by a user. As the actuation member 1430 is inverted, the extension arm 1462 of the retention element 1460 moves outward and away from the biasing element 1450, releasing the biasing element 1450 from the retention ledge 1462. In some variations, the actuation member 1430 remains inverted, decreasing the profile (e.g., the height) of the wearable analyte monitoring device 1400. In some variations, the actuation member 1430 may return to its original shape.

In some variations, a second biasing element may be arranged in the cavity of the housing body to provide additional compression of the microneedle array 140 once inserted into the skin of the user. For example, the second biasing element may be positioned in a volume defined between the housing body and the biasing element 1450. The second biasing element may be a spring, such as a coiled compression spring. The second biasing element may be in a first compressed state when the biasing element 1450 is in the loaded configuration and in a second compressed state when the biasing element 1450 is in the deployed configuration. The second compressed state may provide an additional force on the biasing element 1450 while in the deployed configuration.

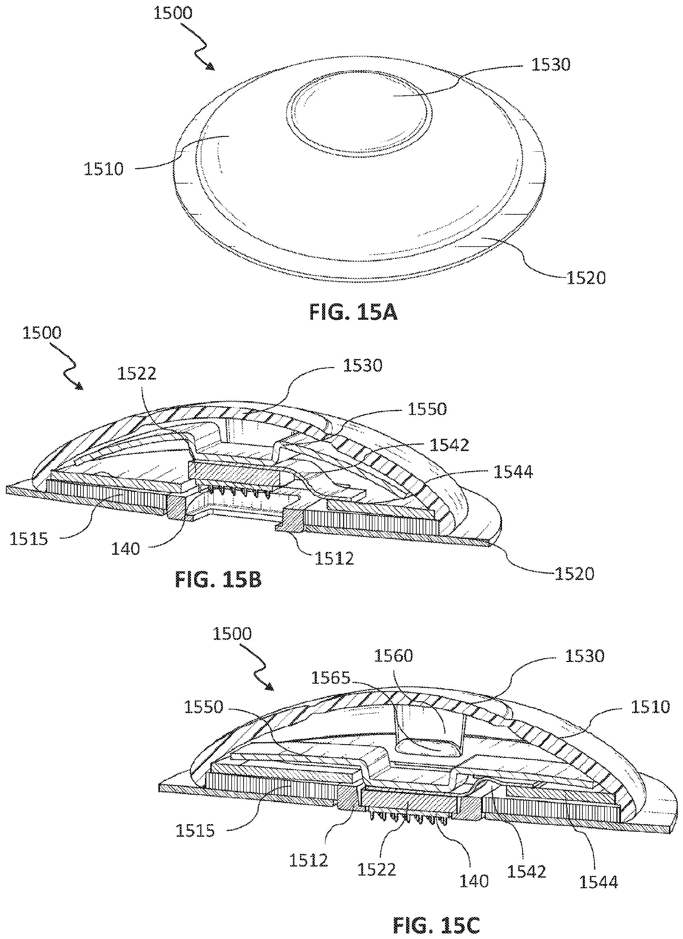

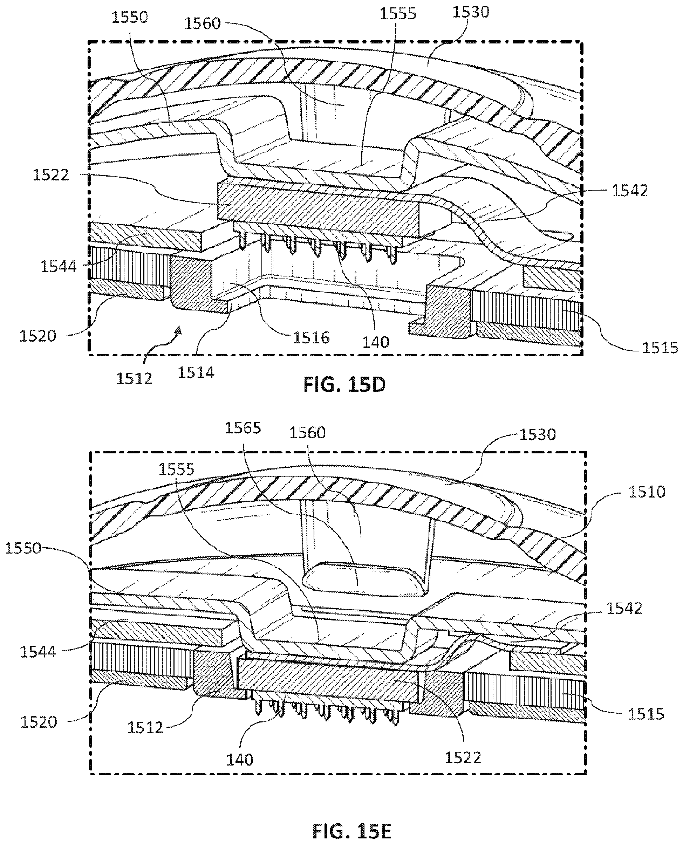

FIGS. 15A-15E depict aspects of a wearable analyte monitoring device with integrated applicator 1500, according to some variations. FIG. 15A provides an upper perspective view of the analyte monitoring device with integrated applicator 1500. FIG. 15B, FIG. 15C, FIG. 15D, and FIG. 15E illustrate internal aspects of the analyte monitoring device 1500. FIG. 15B is a side cross-sectional view of the analyte monitoring device 1500 in a loaded configuration for deploying the microneedle array 140 of the analyte monitoring device 1500. FIG. 15C is a side cross-sectional view of the analyte monitoring device 1500 in a deployed configuration. FIG. 15D is a detailed view of the analyte monitoring device 1500 in a loaded configuration for deploying the microneedle array 140. FIG. 15E is a detailed view of the analyte monitoring device 1500 in a deployed configuration.

The analyte monitoring device 1500 includes a housing body comprising an internal cavity wherein various components of the analyte monitoring device 1500 are retained. In some variations, the housing includes a cover 1510 and a housing base 1515 that together form a housing body and define an internal cavity. An adhesive layer 1520 may be provided on a distal end of the housing body (e.g., a bottom, outer-facing region of the housing base 1515) to adhere the analyte monitoring device 1500 to the skin of the user.

In some variations, an actuation member 1530 is formed at a top surface of the housing cover 1510. The actuation member 1530 is a depressible or releasable (e.g., flexible) member that responds to user force. For example, when the user pushes downward on the actuation member 1530, the actuation member responds by depressing inward. After removal of the user force, the actuation member 1530 may assume its original shape. In some variations, the actuation member 1530 may be a deformable portion of the housing cover 1510. For example, the actuation member 1530 may be made of a material that responds to force and/or pressure. Surrounding portions of the housing cover 1510, in some variations, may be made of a stronger, more resilient material that maintains its shape and structure as the actuation member 1530 deforms upon a force applied by a user.

As shown in FIGS. 15B-15E, a biasing element 1550 is arranged in the cavity of the housing of the analyte monitoring device 1500. The biasing element 1550 is coupled to or otherwise connected to the microneedle array 140. The biasing element 1550 thus acts as a support structure for the microneedle array 140. In some variations, the biasing element 1500 comprises a flattened or contoured portion 1555 to facilitate attachment to the microneedle array 140. The biasing element 1550 may be a leaf spring anchored at two points to bias the microneedle array 140 toward a skin surface of a user when the analyte monitoring device 1500 is loaded to deploy the microneedle array 140, as shown in FIG. 15B and FIG. 15D. In this position, the microneedle array 140 is retracted inside the cavity defined by the housing cover 1510 and the housing base 1515 and held in place by an engagement of the biasing element 1550 and a retention element 1560.

The biasing element 1550 may be disengaged from the retention element 1560 upon actuation of the actuation member 1530. For example, by applying force or pressure to an outer surface of the actuation member 1530, the retention element 1560 is released from the biasing element 1550. The release or disengagement of the biasing element 1550 and the retention element 1560 causes an accelerating force on the microneedle array 140, causing insertion into the skin surface of the user. As the biasing element 1550 moves from a loaded configuration to a deployed configuration, the biasing element 1550 moves from a loaded, stressed state and thus presses the microneedle array 140 with a force (e.g., between about 15 to about 35 Newtons) into the skin surface when the device 1500 has been applied on a user.

In some variations, the biasing element 1550 has two opposing ends which are coupled, attached, or otherwise anchored to an inside surface of the housing cover 1510, a surface of the main PCB 1544, or a surface of the housing base 1515. During assembly, a middle portion of the biasing element 1550 (which may be configured for attachment to the microneedle array and/or connection component) is translated and engaged with the retention element 1560, thereby providing the biasing element 1550 in the loaded configuration. In the loaded configuration, the biasing element 1550 is provided in a bent, stressed state, such that the middle portion of the biasing element 1550 accelerates the attached microneedle array 140 toward a skin surface when the biasing element 1550 is disengaged from the retention element 1560.

In variations, the retention element 1560 is integral with the actuation member 1530 and/or coupled thereto. The retention element 1560 may include a retention ledge 1565. In some variations, the retention ledge 1565 provides a support surface for the biasing element 1550 or an outer edge of the biasing element 1550 to rest on, interface with, and/or otherwise engage with the retention ledge 1565 to retain the analyte monitoring device 1500 in the loaded configuration. In some variations, the retention ledge engages with connection component 1522 to retain the analyte monitoring device 1500 in the loaded configuration.

In some variations, a second biasing element (not shown) may be arranged in the cavity of the housing to provide additional compression of the microneedle array 140 once inserted into the skin of the user. For example, the second biasing element may be positioned in a volume defined between the housing and the biasing element 1550. The second biasing element may be a spring, such as a coiled compression spring. The second biasing element may be in a first compressed state when the biasing element 1550 is loaded and in a second compressed state when the biasing element 1550 is deployed. The second compressed state may provide an additional compressive force on the microneedle array 140 while transitioning the microneedle array 140 from the first configuration to the second configuration.

In some variations, the analyte monitoring device 1500 comprises a printed circuit board (PCB) assembly, including a main PCB portion 1544 and a flexible PCB portion 1542, arranged in the housing (e.g., in a cavity defined by the housing cover 1510 and the housing base 1515). The flexible PCB 1542 may be configured to connect to the microneedle array 140 array to the main PCB 1544 to allow movement of the microneedle array 140 relative to the main PCB 1544 while maintaining an electrical connection. In some variations, the main PCB portion 1544 is also a flexible printed circuit board. The main PCB portion 1544 and the flexible PCB portion 1542 may therefore be integrated and do not require a connection to be established between them. In some variations, the microneedle array 140 may be electrically connected to the flexible PCB 1542 through, for example, a connection component 1522. The connection component 1522 may be analogous to the secondary PCB component and/or secondary PCB connector described above (e.g., secondary PCB 420 and secondary PCB connector 430 depicted in FIG. 4B and FIG. 4G), such that the connection component 1522 provides an electrical connection between the electrical contacts on the backside of the microneedle array 140 and the flexible PCB 1542 of the printed circuit board assembly.

In some variations, the housing base 1515, adhesive layer 1520, and/or main PCB 1544 comprise an aperture forming a distal opening of the housing body to allow at least a portion of the microneedle array 140 to extend outwardly from the device. During deployment (transition from the first configuration to the second configuration), a portion of the microneedle array 140 may be translated from within the cavity through the distal opening, such that the microneedles extend from the housing body and penetrate through a skin surface of a user. In some variations, a sealing element is provided, such that the internal cavity is sealed when the microneedle array 140 is deployed. The sealing element may provide a water resistant or waterproof seal to prevent the ingress of moisture into the internal cavity of the housing.

FIG. 15B, FIG. 15C, FIG. 15D, and FIG. 15E, depict a sealing element 1512 comprising a ledge 1514 and an inner wall 1516. In some variations, in a deployed configuration (as depicted in FIG. 15C and FIG. 15E) connection component 1522, coupled to the microneedle array 140, contacts the sealing element 1512 to seal the distal opening, thereby sealing the internal cavity of the housing. In some variations, the connection component 1522 abuts the ledge 1514, the inner wall 1516, or both, of the sealing element 1512 to create a seal. In some variations, an outer edge of the microneedle array 140 abuts the ledge 1514, the inner wall 1516, or both, of the sealing element 1512 to create a seal. In some variations, the inner wall 1516 of the sealing element 1512 is tapered to facilitate an interference, pressed, or friction fit between the sealing element 1512 and the connection component 1522 and/or the microneedle array 140. The sealing element 1512 may be formed of silicone, waterproof polymer, rubber, or similar material suitable for creating a waterproof seal.

In some variations, the sealing element 1512 is integral with the housing base 1515. In some variations, the sealing element 1512 is adhered to or otherwise coupled to the housing base 1515. While the sealing element 1512 is depicted as substantially rectangular or square, the features of the sealing element 1512 may substantially correspond to the shape of the microneedle array 140 and/or connection component 1522. For example, if the microneedle array 140 and/or connection component 1522 are substantially circular, the inner wall 1516 and the ledge 1514 of the sealing element 1512 may also be substantially circular and dimensioned to create an interference fit.

In some variations, the components of the analyte monitoring device may have a conformal waterproof coating to prevent corrosion, disruption, or other negative effects resulting from exposure to liquids or moisture. A seal may also be provided by a flexible and/or bellowed membrane. For example, a bellowed membrane may be provided between the microneedle array and the base of the housing, such that moisture is not allowed to pass there between. Such a configuration may allow for the microneedle array to move relative to the housing (e.g., during transition from a first configuration to a second configuration) while maintaining a waterproof seal and preventing moisture from entering the internal cavity of the housing.

In some variations, the biasing element applies a constant force on the microneedle array in the second configuration to retain the microneedles in the skin surface of the user. In some variations, a locking mechanism is utilized to maintain the position of the microneedle array once deployed. For example, the ledge 1514 of the sealing element 1512 may be coated with a contact adhesive such that an outer edge of the microneedle array 140 and/or the connection component 1522 adheres the ledge 1514 of the sealing element 1512 when the ledge 1514 is contacted during deployment. Additional or alternative lockout mechanisms may be utilized, such as detents, spring loaded slides, etc. For example, the retention element 1560 may have a bottom portion, extending beyond the retention ledge 1565, such that the bottom surface of the retention element 1560 abuts the top surface of the biasing element 1550 in the deployed configuration. In such an example, the retention element 1560 may move outwardly during actuation to allow the biasing element 1550 to transition into the deployed configuration, then move back into place after an actuation force has been removed, such that the bottom surface of the retention element abuts the top surface of the biasing element.

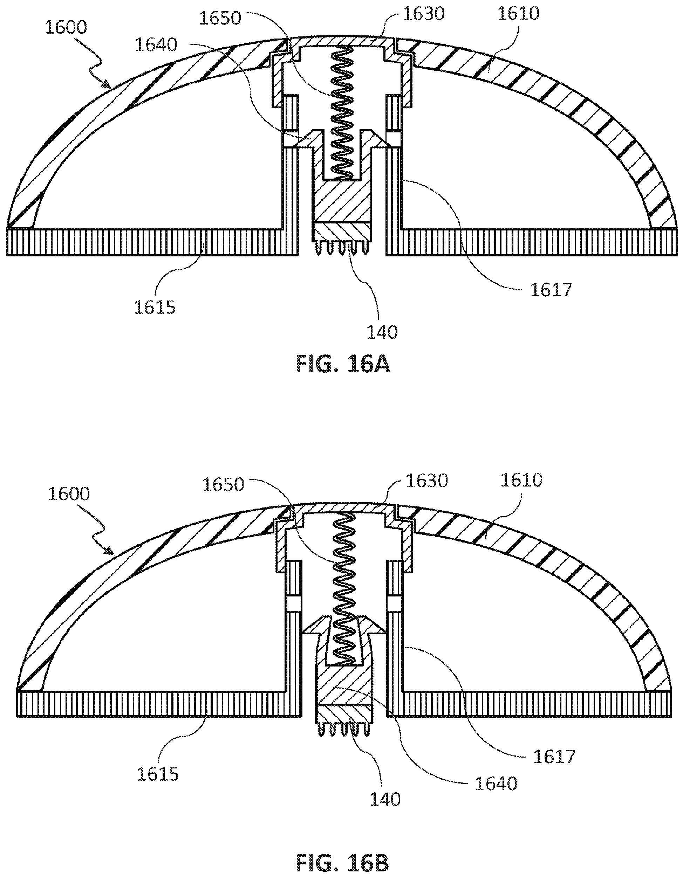

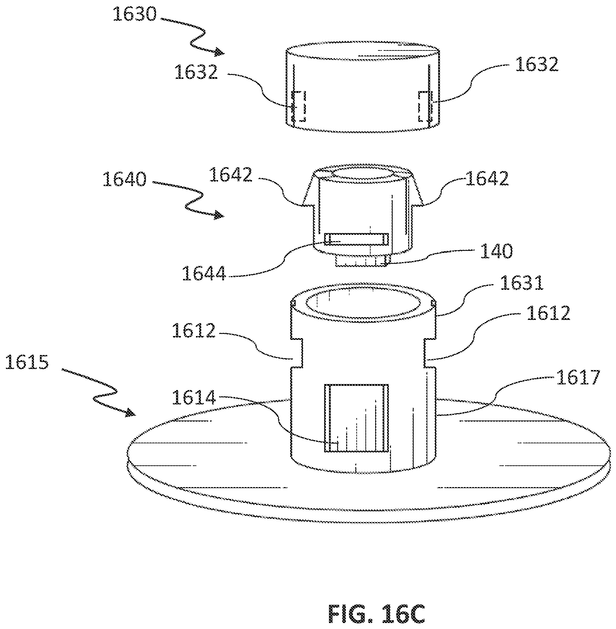

FIG. 16A-16C, depict aspects of a wearable analyte monitoring device with integrated applicator 1600, according to some variations. FIGS. 16A-16C depict a variation of the actuation mechanism for triggering the deployment of a microneedle array 140 configured for monitoring levels of target analytes present in dermal interstitial fluid of a subject. FIG. 16A is a side cross-sectional view of the analyte monitoring device 1600 in a loaded configuration (e.g., when the microneedle array 140 is in a first configuration). FIG. 16B is a side cross-sectional view of the analyte monitoring device 1600 in a deployed configuration (e.g., when the microneedle array 140 is in a second configuration). FIG. 16C is an exploded perspective view of an actuation member 1630, a shuttle 1640, and a housing base 1615 of the analyte monitoring device 1600.

In some variations, the wearable analyte monitoring device 1600 includes a base 1615 having a protrusion 1617. In some variations, the protrusion 1617 is cylindrical and retains a shuttle 1640 when in the loaded configuration (as depicted in FIG. 16A). In some variations, the protrusion 1617 has an inner diameter and an outer diameter and is substantially tubular. The protrusion 1617 extends from a proximal surface of the base 1615 into a cavity formed by the base 1615 and a housing 1610.

In some variations, the shuttle 1640 is a substantially cylindrical member having one or more flexible arms 1642 extending from outer sidewalls thereof. The flexible arms 1642 enable retention of the shuttle 1640 as a distal surface (e.g., protrusions) of the flexible arms 1642 abuts a distal surface of corresponding apertures 1612 of the protrusion 1617 when the analyte monitoring device 1600 is in the loaded configuration (e.g., as shown in FIG. 16A). The microneedle array 140 is coupled to the shuttle 1640 at a distal end thereof such that the microneedles of the microneedle array 140 extend in a distal direction from the distal end of the shuttle 1640. A biasing element 1650 (e.g., a compression spring) may bias the shuttle 1640 and the microneedle array 140 toward the base 1615 of the analyte monitoring device 1600 and away from an actuation member 1630.

In some variations, during deployment of the microneedle array 140, the protrusions of the flexible arms 1642 of the shuttle 1640 are depressed inward by an inner surface of the actuation member 1630, thereby releasing the flexible arms 1642 from engagement with apertures 1612 and allowing the shuttle 1640 and attached microneedle array 140 to be translated toward the base and to a skin surface of a user. In some variations, as described above, the base 1615 comprises an aperture which forms a distal opening of the housing body to allow a plurality of microneedles of the microneedle array 140 to pass through and extend from the device in the deployed configuration (as depicted in FIG. 16B). A seal may be provided or formed in the deployed configuration.

In some variations, the actuation member 1630 is integrated with a top portion of the housing 1610. The actuation member 1630 may therefore be engaged by a user depressing a top portion of the housing. In some variations, the actuation member 1630 may be a separate component from the housing 1610. The biasing element 1650 may also serve to provide a bias against the actuation member 1630 to prevent accidental deployment of the microneedle array 140. In some variations, where the actuation member 1630 is engaged by deforming a portion of the housing 1610, the biasing element 1650 may serve to push the housing 1610 back to its original shape after deployment.

In some variations, the actuation member 1630 has one or more protrusions 1632 which snap into apertures 1612 upon actuation to ensure the flexible arms 1642 of the shuttle 1640 are fully retracted into an interior portion of the protrusion 1617 of the base. In some variations, the protrusions 1632 of the actuation member 1630 are provided on flexible arms to facilitate sliding of the actuation member 1630 over the protrusion 1617 of the base. In some variations, the protrusion 1617 of the base 1615 includes one or more slots or tracks 1631 for guiding the actuation member 1630 and/or the shuttle 1640 as they are translated during actuation and deployment.

While FIGS. 16A-16C depict variations of a shuttle 1640 having two flexible arms 1642 and two corresponding apertures 1612 provided by the protrusion 1617 of the base 1615, it should be appreciated that the number of flexible arms and corresponding apertures may be varied. For example, the shuttle may have one, two, three, four, five, six, or more flexible arms, and the protrusion of the base may include a corresponding number of apertures. Further, the sizing of the flexible arms and corresponding apertures may be varied.

In some variations, the microneedle array 140 is coupled to the shuttle 1640 and the shuttle 1640 is coupled to the biasing element 1650 (e.g., a coil spring), thereby facilitating indirect coupling of the microneedle array 140 to the biasing element. In some variations, electronic components (e.g., a battery, wireless transceiver, microprocessor, etc.) of the wearable analyte monitoring device 1600 are coupled to and/or provided within the shuttle 1640. In some variations, the electronic components are provided elsewhere in the cavity formed by the housing or attached to the base 1615 and connected to the microneedle array 140 by a flexible PCB or wire array. An aperture 1644 provided through the shuttle 1642 may correspond to a slot 1614 formed in the protrusion 1617 of the base 1615 to allow a flexible PCB or wire connection to be maintained during translation of the shuttle 1640 and microneedle array 140 from the first configuration to the second configuration.

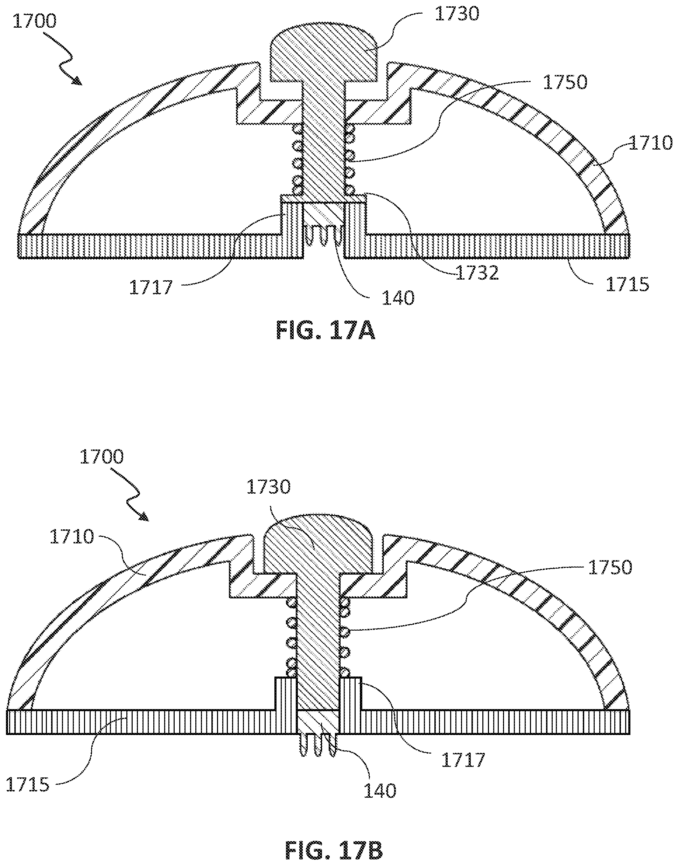

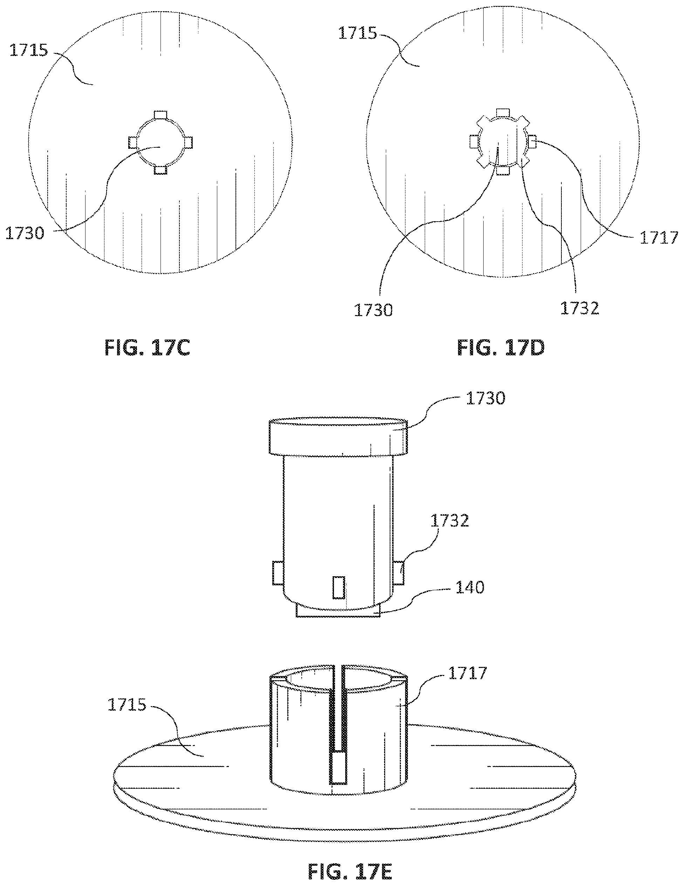

FIGS. 17A -17E depict aspects of a wearable analyte monitoring device with integrated applicator 1700, according to some variations. FIGS. 17A-17E depict a variation of an actuation mechanism for triggering deployment of a microneedle array 140 configured for monitoring levels of target analytes present in dermal interstitial fluid of a subject. FIG. 17A is a side cross-sectional view of the analyte monitoring device 1700 in a loaded configuration (e.g., when the microneedle array 140 is in a first configuration). FIG. 17B is a side cross-sectional view of the analyte monitoring device 1700 in a deployed configuration (e.g., when the microneedle array 140 is in a second configuration). FIG. 17C is a top plan view of the actuation member 1730 and housing base 1715 of the analyte monitoring device 1700 in a loaded configuration (e.g., when the microneedle array 140 is in a first configuration). FIG. 17D is a top plan view of the actuation member 1730 and housing base 1715 of the analyte monitoring device 1700 in a deployed configuration (e.g., when the microneedle array 140 is in a second configuration). FIG. 17E is an exploded perspective view of the actuation member 1730, microneedle array 140, and housing base 1715 of the analyte monitoring device 1700.

In some variations, the wearable analyte monitoring device 1700 has a base 1715 having one or more protrusions 1717. The microneedle array 140 may be coupled to an actuation member 1730. A biasing element 1750 (e.g., a coil spring) may bias the actuation member 1730 and the microneedle array 140 toward the base 1715. In some variations, an actuation member 1730 may have one or more protrusions 1732 which provide a retention element as a bottom surface of protrusions 1732 on a distal surface thereof that abut a proximal surface of the protrusions 1717 of the base 1715 in the loaded configuration (as depicted in FIG. 17C). In some variations, a top portion of the actuation member 1730 is provided external to the housing and is rotatable by a user. To deploy the microneedle array 140, the actuation member 1730 is rotated such that the protrusions 1732 are positioned into slots or spaces provided between the protrusions 1717 of the base 1715 (as depicted in FIG. 17D), thereby releasing the actuation member 1730 and attached microneedle array 140 to be translated toward the base1715 such that the microneedle array 140 protrudes through a distal opening and into a skin surface of a user under influence of a biasing element 1750. In some variations, as described above, the base 1715 comprises a distal opening formed by an aperture to allow a plurality of microneedles of the microneedle array 140 to pass through and extend from the device in the second configuration. A seal may be provided or formed around the microneedle array 140.

While FIGS. 17C-17E depict variations of an actuation member 1730 having four protrusions 1732 and four corresponding protrusions 1717 provided on the base 1715, it should be appreciated that the number of protrusions of the actuation member and corresponding protrusions of the base may be varied. For example, the actuation member may have one, two, three, four, five, six, or more protrusions, and the base may have a corresponding number of protrusions. Further, the sizing of the protrusions may be varied. For example, as depicted in FIG. 17E, larger protrusions with a smaller spacing therebetween provided on the base may form tracks or slots which facilitate guiding and alignment of the microneedle array 140 during deployment.

In some variations, the biasing element 1750 abuts an inner surface of a top portion of the housing 1710 at a first end. In some variations, the biasing element 1750 abuts a top surface of one or more of the protrusions 1732 of the actuation member 1730 at a second end, opposite of the first end. In some variations, the inner surface of the protrusions 1717 of the base 1715 form a guide for the actuation member 1730 during translation. In some variations, the biasing element 1750 is coiled around a portion of the actuation member 1730, and an outer circumference of the biasing element 1750 fits within the inner surface of the protrusions 1717 of the base 1715. In some variations, translation of the actuation member 1730 is stopped when the protrusions 1732 abut a portion of the base 1715, or when a bottom surface of a top portion of the actuation member abuts the housing 1710.

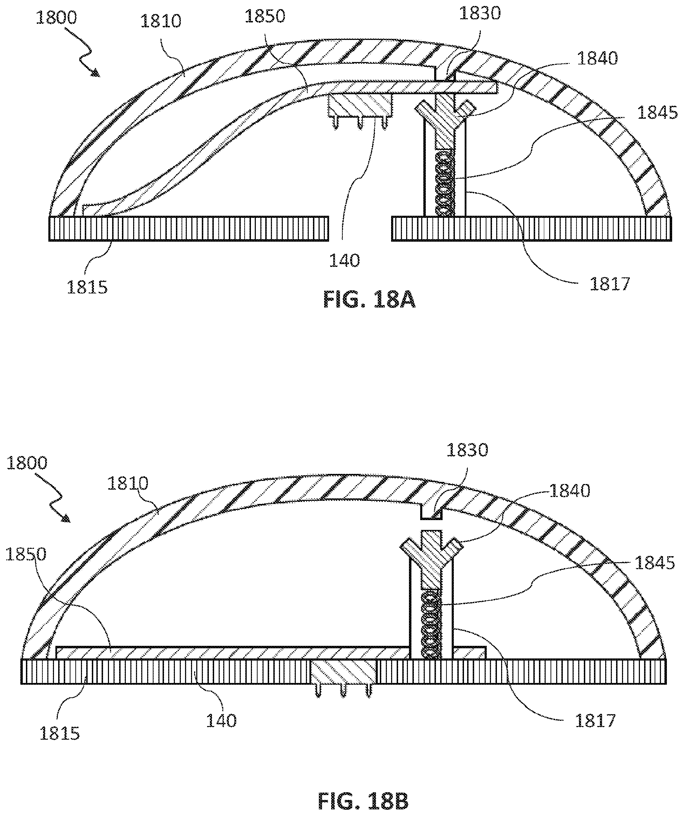

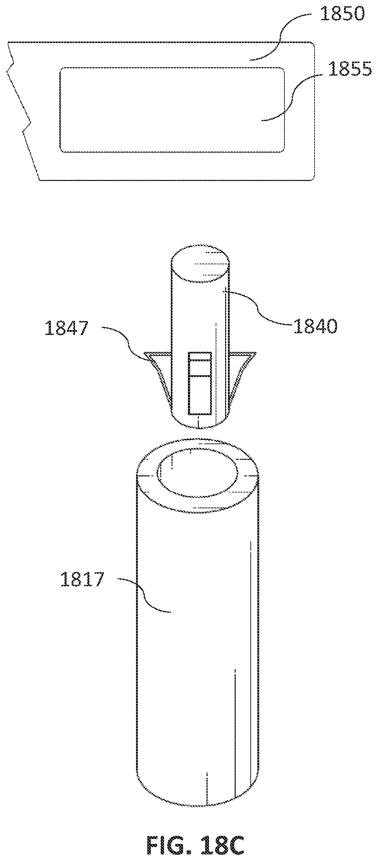

FIGS. 18A -18C depict aspects of a wearable analyte monitoring device with integrated applicator 1800, according to some variations. FIGS. 18A-18C depict a variation of an actuation mechanism for triggering the deployment of a microneedle array 140 configured for monitoring levels of target analytes present in dermal interstitial fluid of a subject. FIG. 18A is a side cross-sectional view of the analyte monitoring device 1800 in a loaded configuration (e.g., when the microneedle array 140 is in a first configuration). FIG. 18B is a side cross-sectional view of the analyte monitoring device 1800 in a deployed configuration (e.g., when the microneedle array 140 is in a second configuration). FIG. 18C is an exploded perspective view of the biasing element 1850, retention element 1840, and protrusion of the base 1817 of the analyte monitoring device 1800.

In some variations, the wearable analyte monitoring device 1800 has a base 1815 with a protrusion 1817. The microneedle array 140 may be coupled to a biasing element 1850. The biasing element 1850 (e.g., a leaf spring) may bias the microneedle array 140 toward the base 1815 of the device 1800. In some variations, a retention element 1840 fits within the protrusion of the base 1817 and has one or more flexible wings 1847. The biasing element 1850 may be attached to, or anchored at, the first end. The second end of the biasing element 1850 may include a slot 1855 having a width slightly greater than the outer diameter of the protrusion 1817 of the base 1815. In the loaded configuration (as depicted in FIG. 18A), the retention element may be biased away from the base by spring 1845, such that wings 1847 are outside of the protrusion 1817 and extend outward from the body of the retention element 1840. In the loaded configuration, wings 1847 extend beyond the width of the slot 1855 of the biasing element 1850, such that a bottom surface of the biasing element 1850 abuts the wings 1847, and the wings 1847 abut the protrusion. In some variations, engagement of the actuation member 1830 pushes the retention element 1840 into the protrusion 1817 and the wings 1847 are depressed inward as the retention element 1840 is forced into the protrusion. The inward depression of the wings 1847 releases the biasing element 1850, as the wings 1847 no longer impede the translation of the biasing element. As the biasing element 1850 is translated toward the base 1815, the protrusion 1817 moves through the slot 1855 of the biasing element and the microneedle array 140 is deployed (as depicted in FIG. 18B).

While FIG. 18C depicts a variation of the retention element 1840 having four wings 1847, it should be appreciated that the number of wings may be varied. For example, the retention element may have one, two, three, four, five, six, or more flexible wings. Further, the sizing of the flexible wings may be varied.

In some variations, the actuation member 1830 includes a flexible portion of the housing 1810 which is depressed to abut the retention element 1840. In some variations, the actuation member 1830 has a protrusion to abut the retention element 1840. The protrusion may be coupled to or integrated to a flexible portion of the housing 1810 which is depressed by a user. In some variations, the retention element 1840 is coupled to or integrated with the actuation member 1830. As described above, the base 1815 may comprise an aperture, which forms a distal opening of the housing body, to allow a plurality of microneedles of the microneedle array 140 to pass through and extend from the device in the second configuration. A seal may be provided or formed in the deployed configuration.

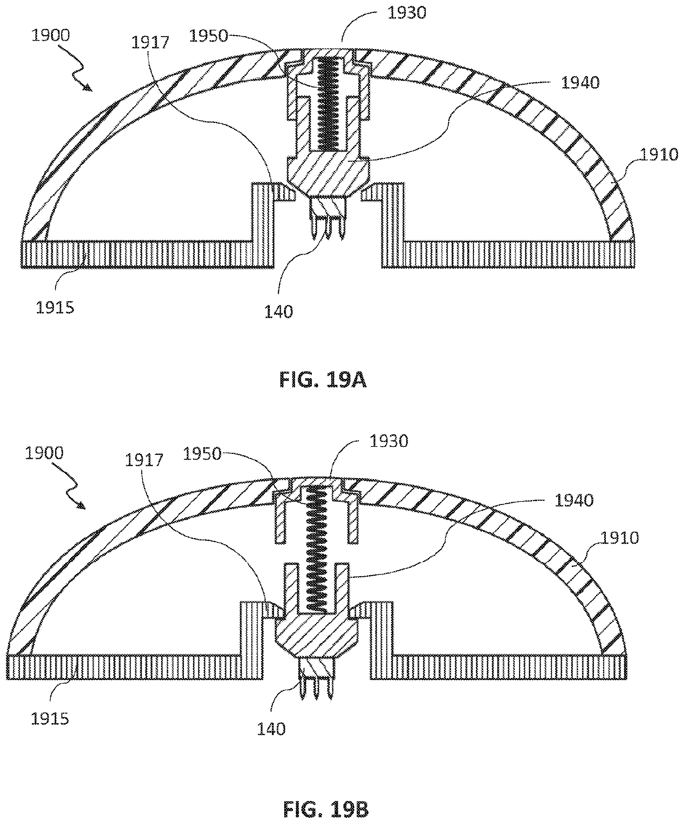

FIGS. 19A -19B depict aspects of a wearable analyte monitoring device with integrated applicator 1900, according to some variations. FIGS. 19A-19B depict a variation of an actuation mechanism for triggering the deployment of a microneedle array 140 configured for monitoring levels of target analytes present in dermal interstitial fluid of a subject. FIG. 19A is a side cross-sectional view of the analyte monitoring device 1900 in a loaded configuration (e.g., when the microneedle array 140 is in a first configuration). FIG. 19B is a side cross-sectional view of the analyte monitoring device 1900 in a deployed configuration (e.g., when the microneedle array 140 is in a second configuration).

In some variations, the wearable analyte monitoring device 1900 has a base 1915 having one or more protrusions 1917. The protrusions 1917 extend from a proximal surface of a base 1915 into a cavity formed by the base 1915 and a housing 1910. In some variations, the protrusions 1917 form a retaining element to hold a shuttle 1940 when in a loaded configuration (as depicted in FIG. 19A). The microneedle array 140 may be coupled to the shuttle 1940 (e.g., coupled to a distal end of the shuttle 1940). A biasing element 1950 (e.g., a compression spring) may bias the shuttle 1940 and the microneedle array 140 toward the base 1915 of the device and away from an actuation member 1930. In the loaded configuration, movement of the shuttle 1940 and the microneedle array 140 is prevented by one or more opposing surfaces of the protrusions 1917. For example, the protrusions 1917 form a stop that prevents vertical translation of the shuttle 1940 and the microneedle array 140 in a distal direction.

In some variations, a user engages the actuation member 1930 such that one or more surfaces of the actuation member 1930 abut the shuttle 1940. The depression applied to the actuation member 1930 is translated to the shuttle 1940 to force a portion of shuttle 1940 into an opening created by the protrusions 1917. In some variations, the shuttle 1940 is tapered to facilitate unidirectional passing of the shuttle 1940 through the protrusions 1917. After the distal end of the shuttle 1940 passes through opening created by the protrusions 1917, the biasing element 1950 translates the microneedle array 140 to the second configuration (as depicted in FIG. 19B). In some variations, a depth of the microneedle array 140 is locked or secured once the shuttle 1940 passes through the protrusions 1917. The thickness or depth of the proximal portion of the shuttle 1940 may be varied to control the insertion depth of the microneedle array 140 in a second configuration.

In some variations, the protrusions 1917 are flexible (e.g., formed from a flexible material) and are deflected outward by the shuttle 1940 upon depression of the actuation member 1930, allowing the shuttle 1940 to pass through into the opening. In some variations, the shuttle 1940 has one or more flexible members which are deflected inward by the protrusions 1917 upon depression of the actuation member 1930, allowing the shuttle 1940 to pass through. The abutting surfaces of the shuttle 1940 and/or protrusions 1917 may be beveled (e.g., ramped, tapered, etc.) to facilitate translation of the shuttle 1940 past the protrusions 1917. While FIGS. 19A-19B depict variations of a base having two flexible protrusions 1917, it should be appreciated that the number of protrusions may be varied. For example, the base may have one, two, three, four, five, six, or more protrusions. Further, the sizing and spacing of the protrusions may be varied. In some variations, the protrusion 1917 is substantially annular and flexible (e.g., formed from a flexible material). In some variations, a proximal end of the protrusion 1917 is flexible to facilitate passage of the shuttle upon application of a force by a user on the actuation member 1930.

In some variations, the actuation member 1930 is integrated with a top portion of the housing 1910. The actuation member 1930 may therefore be engaged by a user depressing a top portion of the housing. In some variations, the actuation member 1930 may be a separate component from the housing 1910. The biasing element 1950 may also serve to provide a bias against the actuation member 1930 to prevent accidental deployment of the microneedle array 140. In some variations, where the actuation member 1930 is engaged by deforming a portion of the housing 1910, the biasing element 1950 may serve to push the housing 1910 back to its original shape after deployment. In some variations, protrusions 1917 define distal opening in the base 1915 to allow a plurality of microneedles of the microneedle array 140 to pass through and extend from the analyte monitoring device 1900 when in the deployed configuration. As described above, a seal may be provided or formed in the deployed configuration. In some variations, a seal is provided between a distal portion of the shuttle 1940 and an inner surface formed by the protrusions 1917.

In some variations, one or more electrical connections are established when the microneedle array reaches the second configuration (e.g., the microneedle array is deployed). As disclosed above, features of the device (e.g., a flexible PCB connection) may allow the microneedle array to maintain an electrical connection with a main PCB as it moves from the first configuration to the second configuration. Further, additional electrical connections may be established when the microneedle array 140 is in the second configuration (e.g., when the microneedle array 140 is deployed). For example, one or more electrical contacts may be provided to provide an open circuit in the first configuration and establish a closed circuit in the second configuration. Establishing new electrical connections in the second configuration may be utilized to power on the components of the analyte monitoring device, establish a connection to a battery of the analyte monitoring device, wake the analyte monitoring device from a sleep state, and/or transition the analyte monitoring device from a low-power mode to a full-power mode.

In some variations, some or all components of the analyte monitoring system may be provided in a kit (e.g., to a user, to a clinician, etc.). For example, a kit may include at least one analyte monitoring device. In some variations, a kit may include multiple analyte monitoring devices, which may form a supply of analyte monitoring devices sufficient that is for a predetermined period of time (e.g., a week, two weeks, three weeks, a month, two months, three months, six months, a year, etc.).

In some variations, the kit may further include user instructions for operating the analyte monitoring device and/or applicator (e.g., instructions for applying the analyte monitoring device manually or with the applicator, instructions for pairing the analyte monitoring device with one or more peripheral devices (e.g., computing devices such as a mobile phone), etc.).

Described below is an overview of various aspects of a method of use and operation of the analyte monitoring system, including the analyte monitoring device and peripheral devices, etc.

As described above, the analyte monitoring device is applied to the skin of a user such that the microneedle array in the device penetrates the skin and the microneedle array's electrodes are positioned in the upper dermis for access to dermal interstitial fluid. For example, in some variations, the microneedle array may be geometrically configured to penetrate the outer layer of the skin, the stratum corneum, bore through the epidermis, and come to rest within the papillary or upper reticular dermis. The sensing region, confined to the electrode at the distal extent of each microneedle constituent of the array (as described above) may be configured to rest and remain seated in the papillary or upper reticular dermis following application in order to ensure adequate exposure to circulating dermal interstitial fluid (ISF) without the risk of bleeding or undue influence with nerve endings.

In some variations, the analyte monitoring device may include a wearable housing or patch with an adhesive layer provided at a distal end of the housing and configured to adhere to the skin and fix the microneedle array in position.

The analyte monitoring device may be applied in any suitable location, though in some variations it may be desirable to avoid anatomical areas of thick or calloused skin (e.g., palmar and plantar regions), or areas undergoing significant flexion (e.g., olecranon or patella). Suitable wear sites may include, for example, on the arm (e.g., upper arm, lower arm, forearm, or volar forearm), shoulder (e.g., over the deltoid), back of hands, neck, face, scalp, torso (e.g., on the back such as in the thoracic region, lumbar region, sacral region, etc. or on the chest or abdomen), buttocks, legs (e.g., upper legs, lower legs, etc.), and/or top of feet, etc.

Once the analyte monitoring device is inserted and warm-up and any calibration has completed, the analyte monitoring device may be ready for providing sensor measurements of a target analyte. The target analyte (and any requisite co-factor(s)) diffuses from the biological milieu, through the biocompatible and diffusion-limiting layers on the working electrode, and to the biorecognition layer including the biorecognition element. In the presence of a co-factor (if present), the biorecognition element may convert the target analyte to an electroactive product.

A bias potential may be applied between the working and reference electrodes of the analyte monitoring device, and an electrical current may flow from the counter electrode to maintain the fixed potential relationship between the working and reference electrodes. This causes the oxidation or reduction of the electroactive product, causing a current to flow between the working electrodes and counter electrodes. The current value is proportional to the rate of the redox reaction at the working electrode and, specifically, to the concentration of the analyte of interest according to the Cottrell relation as described in further detail above.

The electrical current may be converted to a voltage signal by a transimpedance amplifier and quantized to a digital bitstream by means of an analog-to-digital converter (ADC). Alternatively, the electrical current may be directly quantized to a digital bitstream by means of a current-mode ADC. The digital representation of the electrical current may be processed in the embedded microcontroller(s) in the analyte monitoring device and relayed to the wireless communication module for broadcast or transmission (e.g., to one or more peripheral devices). In some variations, the microcontroller may perform additional algorithmic treatment to the data to improve the signal fidelity, accuracy, and/or calibration, etc.

In some variations, the digital representation of the electrical current, or sensor signal, may be correlated to an analyte measurement (e.g., glucose measurement) by the analyte monitoring device. For example, the microcontroller may execute a programmed routine in firmware to interpret the digital signal and perform any relevant algorithms and/or other analysis. Keeping the analysis on-board the analyte monitoring device may, for example, enable the analyte monitoring device to broadcast analyte measurement(s) to multiple devices in parallel, while ensuring that each connected device has the same information. Thus, generally, the user's target analyte (e.g., glucose) values may be estimated and stored in the analyte monitoring device and communicated to one or more peripheral devices.

The foregoing description, for purposes of explanation, used specific nomenclature to provide a thorough understanding of the invention. However, it will be apparent to one skilled in the art that specific details are not required in order to practice the disclosure Thus, the foregoing descriptions of specific embodiments of the invention are presented for purposes of illustration and description. They are not intended to be exhaustive or to limit the invention to the precise forms disclosed; obviously, many modifications and variations are possible in view of the above teachings. The embodiments were chosen and described in order to explain the principles of the invention and its practical applications, they thereby enable others skilled in the art to utilize the invention and various embodiments with various modifications as are suited to the particular use contemplated. It is intended that the following claims define the scope of the invention.

## Claims

1. A wearable analyte monitoring device (110), comprising:
a housing (112) comprising a body defining a cavity therein, wherein the housing body comprises a distal opening (334) and a deformable portion (1430), the deformable portion being an actuation member (1430);
an adhesive layer (342; 1520) coupled to a distal end of the housing and surrounding the distal opening, the adhesive layer configured to secure the device to a skin surface of the user;
a retention element (1460; 1560) comprising an extension arm (1462) and a retention ledge (1464), the retention element contained within the cavity and coupled to the actuation member;
a biasing element (1450) contained with the cavity and comprising an edge configured to removably engage with the retention ledge of the retention element; and
a microneedle array (140; 510; 1100; 1200; 1300A; 1300B) coupled to the biasing element and comprising a plurality of microneedles, wherein application of force to the actuation member moves the actuation member inward, resulting in outward flexing of the extension arm and movement of the microneedle array between a first configuration and a second configuration, and wherein:
in the first configuration, the edge of the biasing element engages the retention ledge, holding the microneedle array within the cavity of the housing body, and
in the second configuration, the edge of the biasing element is released from the retention ledge, causing the microneedle array to protrude through the distal opening of the housing body.

2. The wearable analyte monitoring device of claim 1, wherein in the second configuration, the plurality of microneedles is inserted through the skin surface of the user.

3. The wearable analyte monitoring device of claim 1, wherein a seal is formed between an outer perimeter of the microneedle array and the distal opening when the microneedle array is in the second configuration.

4. The wearable analyte monitoring device of claim 1, wherein the retention element is integrated with the actuation member of the housing body.

5. The wearable analyte monitoring device of claim 1, wherein the housing body comprises one or more tapered portions to facilitate flexing of the actuation member upon application of the force.

6. The wearable analyte monitoring device of claim 1, wherein the actuation member retains its shape in response to the application of pressure.

7. The wearable analyte monitoring device of claim 1, wherein the actuation member returns to its original shape after the applied force is removed.

8. The wearable analyte monitoring device of claim 1, wherein the biasing element comprises a leaf spring, the leaf spring configured to maintain a compressive force on the microneedle array when the microneedle array protrudes through the distal opening of the housing body.

9. The wearable analyte monitoring device of claim 1, further comprising a printed circuit board contained within the cavity of the housing body.

10. The wearable analyte monitoring device of claim 9, wherein the printed circuit board comprises a flexible portion coupled to the biasing element, wherein the microneedle array is mounted on the flexible portion of the printed circuit board.

11. The wearable analyte monitoring device of claim 10, wherein the flexible portion of the printed circuit board moves with the biasing element and the microneedle array from the first configuration to the second configuration.

12. The wearable analyte monitoring device of claim 9, wherein the biasing element comprises a flexible portion of the printed circuit board.

13. The wearable analyte monitoring device of claim 1, further comprising a second biasing element contained in the cavity of the housing body, wherein the second biasing element is in a first compressed state in the first configuration and a second compressed state in the second configuration, wherein the second biasing element is configured to provide a force on the biasing element in the second configuration.

14. The analyte monitoring device of claim 13, wherein the second biasing element comprises a coiled compression spring.

15. A method comprising:
providing the wearable analyte monitoring device of claim 1;
adhering the wearable analyte monitoring device to the skin surface of the user; and
transitioning, by applying force to the actuation member, the microneedle array from the first configuration to the second configuration.

## Patentansprüche

1. Am Körper tragbare Analytüberwachungsvorrichtung (110), umfassend:
ein Gehäuse (112), das einen Körper umfasst, der einen Hohlraum darin definiert, wobei der Gehäusekörper eine distale Öffnung (334) und einen verformbaren Abschnitt (1430) umfasst, wobei der verformbare Abschnitt ein Betätigungselement (1430) ist;
eine Klebeschicht (342; 1520), die mit einem distalen Ende des Gehäuses gekoppelt ist und die distale Öffnung umgibt, wobei die Klebeschicht dazu ausgelegt ist, die Vorrichtung an einer Hautoberfläche des Benutzers zu befestigen;
ein Rückhalteelement (1460; 1560), das einen Verlängerungsarm (1462) und eine Rückhalteleiste (1464) umfasst, wobei das Rückhalteelement innerhalb des Hohlraums enthalten und mit dem Betätigungselement gekoppelt ist;
ein Vorspannelement (1450), das in dem Hohlraum enthalten ist und eine Kante umfasst, die dazu ausgelegt ist, entfernbar in die Rückhalteleiste des Rückhalteelements einzugreifen; und
eine Mikronadelanordnung (140; 510; 1100; 1200; 1300A; 1300B), die mit dem Vorspannelement gekoppelt ist und eine Mehrzahl von Mikronadeln umfasst, wobei eine Anwendung von Kraft auf das Betätigungselement das Betätigungselement nach innen bewegt, was zu einer Auswärtsbiegung des Verlängerungsarms und einer Bewegung der Mikronadelanordnung zwischen einer ersten Konfiguration und einer zweiten Konfiguration führt, und
wobei:
in der ersten Konfiguration die Kante des Vorspannelements in die Rückhalteleiste eingreift und die Mikronadelanordnung innerhalb des Hohlraums des Gehäusekörpers hält, und
in der zweiten Konfiguration die Kante des Vorspannelements von der Rückhalteleiste gelöst wird, wodurch bewirkt wird, dass die Mikronadelanordnung durch die distale Öffnung des Gehäusekörpers vorsteht.

2. Am Körper tragbare Analytüberwachungsvorrichtung nach Anspruch 1, wobei in der zweiten Konfiguration die Mehrzahl von Mikronadeln durch die Hautoberfläche des Benutzers eingeführt ist.

3. Am Körper tragbare Analytüberwachungsvorrichtung nach Anspruch 1, wobei eine Dichtung zwischen einem äußeren Umfang der Mikronadelanordnung und der distalen Öffnung ausgebildet ist, wenn sich das Mikronadelarray in der zweiten Konfiguration befindet.

4. Am Körper tragbare Analytüberwachungsvorrichtung nach Anspruch 1, wobei das Rückhalteelement in das Betätigungselement des Gehäusekörpers integriert ist.

5. Am Körper tragbare Analytüberwachungsvorrichtung nach Anspruch 1, wobei der Gehäusekörper einen oder mehrere sich verjüngende Abschnitte umfasst, um Biegung des Betätigungselements bei Anwendung der Kraft zu erleichtern.

6. Am Körper tragbare Analytüberwachungsvorrichtung nach Anspruch 1, wobei das Betätigungselement seine Form in Reaktion auf die Anwendung von Druck beibehält.

7. Am Körper tragbare Analytüberwachungsvorrichtung nach Anspruch 1, wobei das Betätigungselement in seine ursprüngliche Form zurückkehrt, nachdem die angewendete Kraft entfernt ist.

8. Am Körper tragbare Analytüberwachungsvorrichtung nach Anspruch 1, wobei das Vorspannelement eine Blattfeder umfasst, wobei die Blattfeder dazu ausgelegt ist, eine Druckkraft auf die Mikronadelanordnung aufrechtzuerhalten, wenn die Mikronadelanordnung durch die distale Öffnung des Gehäusekörpers vorsteht.

9. Am Körper tragbare Analytüberwachungsvorrichtung nach Anspruch 1, ferner umfassend eine Leiterplatte, die innerhalb des Hohlraums des Gehäusekörpers enthalten ist.

10. Am Körper tragbare Analytüberwachungsvorrichtung nach Anspruch 9, wobei die Leiterplatte einen flexiblen Abschnitt umfasst, der mit dem Vorspannelement gekoppelt ist, wobei die Mikronadelanordnung auf dem flexiblen Abschnitt der Leiterplatte montiert ist.

11. Am Körper tragbare Analytüberwachungsvorrichtung nach Anspruch 10, wobei sich der flexible Abschnitt der Leiterplatte mit dem Vorspannelement und der Mikronadelanordnung von der ersten Konfiguration in die zweite Konfiguration bewegt.

12. Am Körper tragbare Analytüberwachungsvorrichtung nach Anspruch 9, wobei das Vorspannelement einen flexiblen Abschnitt der Leiterplatte umfasst.

13. Am Körper tragbare Analytüberwachungsvorrichtung nach Anspruch 1, ferner umfassend ein zweites Vorspannelement, das in dem Hohlraum des Gehäusekörpers enthalten ist, wobei sich das zweite Vorspannelement in der ersten Konfiguration in einem ersten komprimierten Zustand befindet und in der zweiten Konfiguration in einem zweiten komprimierten Zustand befindet, wobei das zweite Vorspannelement dazu ausgelegt ist, in der zweiten Konfiguration eine Kraft auf das Vorspannelement bereitzustellen.

14. Analytüberwachungsvorrichtung nach Anspruch 13, wobei das zweite Vorspannelement eine spiralförmige Druckfeder umfasst.

15. Verfahren, umfassend:
Bereitstellen der am Körper tragbaren Analytüberwachungsvorrichtung nach Anspruch 1;
Kleben der tragbaren Analytüberwachungsvorrichtung an die Hautoberfläche des Benutzers; und
Überführen der Mikronadelanaordnung von der ersten Konfiguration in die zweite Konfiguration durch Anwenden von Kraft auf das Betätigungselement.

## Revendications

1. Dispositif de surveillance d'analyte portable (110), comprenant :
un boîtier (112) comprenant un corps définissant une cavité dans celui-ci, dans lequel le corps de boîtier comprend une ouverture distale (334) et une partie déformable (1430), la partie déformable étant un élément d'actionnement (1430) ;
une couche adhésive (342 ; 1520) accouplée à une extrémité distale du boîtier et entourant l'ouverture distale, la couche adhésive étant configurée pour assujettir le dispositif à une surface cutanée de l'utilisateur ;
un élément de retenue (1460 ; 1560) comprenant un bras d'extension (1462) et un rebord de retenue (1464), l'élément de retenue étant contenu dans la cavité et accouplé à l'élément d'actionnement ;
un élément de sollicitation (1450) contenue dans la cavité et comprenant un bord configuré pour venir en prise amovible avec le rebord de retenue de l'élément de retenue ; et
un réseau de micro-aiguilles (140 ; 510 ; 1100 ; 1200 ; 1300A ; 1300 B) accouplé à l'élément de sollicitation et comprenant une pluralité de micro-aiguilles, dans lequel l'application d'une force sur l'élément d'actionnement déplace l'élément d'actionnement vers l'intérieur, entraînant une flexion vers l'extérieur du bras d'extension et un déplacement du réseau de micro-aiguilles entre une première configuration et une seconde configuration, et dans lequel :
dans la première configuration, le bord de l'élément de sollicitation entre en prise avec le rebord de retenue, maintenant le réseau de micro-aiguilles dans la cavité du corps de boîtier, et
dans la seconde configuration, le bord de l'élément de sollicitation est libéré du rebord de retenue, ce qui fait dépasser le réseau de micro-aiguilles à travers l'ouverture distale du corps de boîtier.

2. Dispositif de surveillance d'analyte portable selon la revendication 1, dans lequel, dans la seconde configuration, la pluralité de micro-aiguilles est insérée à travers la surface de la peau de l'utilisateur.

3. Dispositif de surveillance d'analyte portable selon la revendication 1, dans lequel un joint d'étanchéité est formé entre un périmètre extérieur du réseau de micro-aiguilles et l'ouverture distale lorsque le réseau de micro-aiguilles se trouve dans la seconde configuration.

4. Dispositif de surveillance d'analyte portable selon la revendication 1, dans lequel l'élément de retenue est intégré à l'élément d'actionnement du corps de boîtier.

5. Dispositif de surveillance d'analyte portable selon la revendication 1, dans lequel le corps de boîtier comprend une ou plusieurs parties effilées pour faciliter la flexion de l'élément d'actionnement lors de l'application de la force.

6. Dispositif de surveillance d'analyte portable selon la revendication 1, dans lequel l'élément d'actionnement conserve sa forme en réponse à l'application d'une pression.

7. Dispositif de surveillance d'analyte portable selon la revendication 1, dans lequel l'élément d'actionnement reprend sa forme d'origine après que la force appliquée a été supprimée.

8. Dispositif de surveillance d'analyte portable selon la revendication 1, dans lequel l'élément de sollicitation comprend un ressort à lame, le ressort à lame étant configuré pour maintenir une force de compression sur le réseau de micro-aiguilles lorsque le réseau de micro-aiguilles dépasse de l'ouverture distale du corps de boîtier.

9. Dispositif de surveillance d'analyte portable selon la revendication 1, comprenant en outre une carte de circuit imprimé contenue dans la cavité du corps de boîtier.

10. Dispositif de surveillance d'analyte portable selon la revendication 9, dans lequel la carte de circuit imprimé comprend une partie flexible accouplée à l'élément de sollicitation, dans lequel le réseau de micro-aiguilles est monté sur la partie flexible de la carte de circuit imprimé.

11. Dispositif de surveillance d'analyte portable selon la revendication 10, dans lequel la partie flexible de la carte de circuit imprimé passe, avec l'élément de sollicitation et le réseau de micro-aiguilles, de la première configuration à la seconde configuration.

12. Dispositif de surveillance d'analyte portable selon la revendication 9, dans lequel l'élément de sollicitation comprend une partie flexible de la carte de circuit imprimé.

13. Dispositif de surveillance d'analyte portable selon la revendication 1, comprenant en outre un second élément de sollicitation contenu dans la cavité du corps de boîtier, dans lequel le second élément de sollicitation est dans un premier état comprimé dans la première configuration et un second état comprimé dans la seconde configuration, dans lequel le second élément de sollicitation est configuré pour fournir une force sur l'élément de sollicitation dans la seconde configuration.

14. Dispositif de surveillance d'analyte selon la revendication 13, dans lequel le second élément de sollicitation comprend un ressort de compression enroulé.

15. Procédé comprenant :
la fourniture du dispositif de surveillance d'analyte portable selon la revendication 1 ;
la mise à adhérer du dispositif de surveillance d'analyte portable sur la surface cutanée de l'utilisateur ; et
le passage, par application d'une force sur l'organe d'actionnement, du réseau de micro-aiguilles de la première configuration à la seconde configuration.
